# EUROPEAN PATENT APPLICATION

(11) **EP 1 741 776 A1**
(43) Date of publication of application: **10.01.2007**
(21) Application number: 04799738.2
(22) Date of filing: 11.11.2004
(51) Int. Cl.: C12N 5/06

(54) **PROCESS FOR PRODUCING MULTIPOTENTIAL STEM CELL ORIGINATING IN TESTOID CELL**

(30) Priority: 30.03.2004 JP 2004101320
(71) Applicant: Kyoto University, Kyoto-shi, Kyoto 606-8501 (JP)
(72) Inventor: SHINOHARA, Takashi, Sakyo-ku Kyoto-shi Kyoto 6068351 (JP); SHINOHARA, Mito, Sakyo-ku Kyoto-shi Kyoto 6068351 (JP)
(74) Representative: Duckworth, Timothy John
(86) International application number: PCT/JP2004/017125
(87) International publication number: WO 2005/100548

(57) **Abstract**

The present invention provides a method of producing pluripotent stem cells, which comprises culturing testis cells using a medium containing glial cell derived neurotrophic factor (GDNF) or an equivalent thereto to obtain pluripotent stem cells. The medium can further contain leukemia inhibitory factor (LIF), epidermal growth factor (EGF), basic fibroblast growth factor (bFGF) and the like. Using the production method of the present invention, it is possible to produce pluripotent stem cells, which have conventionally been only obtainable from fertilized eggs, embryos and the like, from a postnatal individual. Using the pluripotent stem cells, it is possible, to construct diverse tissues having histocompatibility for autotransplantation, and the pluripotent stem cells are useful in medical fields such as regeneration medicine and gene therapy. Also, the pluripotent stem cells are useful in the field of biotechnology because they can be used to prepare transgenic animals, knockout animals and the like.

SEQUENCE LISTING

<110> Shirankai Kyoto University Faculty of Medicine Alumni
      Association Inc.

<120> Method for producing a pluriipotent stem cell derived from testis
       cells

<130> 09704

<150> JP 2004-101320
<151> 2004-03-30

<160> 18

<170> PatentIn version 3.2

<210> 1
<211> 20
<212> DNA
<213> Artificial

<220>
<223> specific primer for Oct-4

<400> 1
agctgctgaa gcagaagagg                                                20


<210> 2
<211> 20
<212> DNA
<213> Artificial

<220>
<223> specific primer for Oct-4

<400> 2
ggttctcatt gttgtcggct                                                20


<210> 3
<211> 21
<212> DNA
<213> Artificial

<220>
<223> specific primer for UTF1

<400> 3
gatgtcccgg tgactacgtc t                                              21


<210> 4
<211> 21
<212> DNA
<213> Artificial

<220>
<223> specific primer for UTF1

<400> 4
tcggggagga ttcgaaggta t                                              21


<210> 5
<211> 19
<212> DNA
<213> Artificial

<220>
<223> specific primer for HPRT

<400> 5
gctggtgaaa aggacctct                                                 19

<210>  6
<211>  20
<212>  DNA
<213>  Artificial

<220>
<223>  specific primer for HPRT

<400>  6
cacaggacta gaacacctgc                                                    20


<210>  7
<211>  23
<212>  DNA
<213>  Artificial

<220>
<223>  specific primer for H19

<400>  7
ggaatatttg tgtttttgga ggg                                                23


<210>  8
<211>  26
<212>  DNA
<213>  Artificial

<220>
<223>  specific primer for H19

<400>  8
aatttgggtt ggagatgaaa atattg                                             26


<210>  9
<211>  33
<212>  DNA
<213>  Artificial

<220>
<223>  specific primer for Meg3 IG

<400>  9
ggtttggtat atatggatgt attgtaatat agg                                     33


<210>  10
<211>  31
<212>  DNA
<213>  Artificial

<220>
<223>  specific primer for Meg3 IG

<400>  10
ataaaacacc aaatctatac caaaatatac c                                       31


<210>  11
<211>  28
<212>  DNA
<213>  Artificial

<220>
<223>  specific primer for Rasgrf1

<400>  11
gtgtagaata tggggttgtt ttatattg                                           28


<210>  12
<211>  26
<212>  DNA

<213>  Artificial

<220>
<223>  specific primer for Rasgrf1

<400>  12
ataatacaac aacaacaata acaatc                                          26


<210>  13
<211>  26
<212>  DNA
<213>  Artificial

<220>
<223>  specific primer for Igf2r

<400>  13
ttagtggggt atttttattt gtatgg                                          26


<210>  14
<211>  28
<212>  DNA
<213>  Artificial

<220>
<223>  specific primer for Igf2r

<400>  14
aaatatccta aaaatacaaa ctacacaa                                        28


<210>  15
<211>  30
<212>  DNA
<213>  Artificial

<220>
<223>  specific primer for Peg10

<400>  15
gtaaagtgat tggttttgta tttttaagtg                                      30


<210>  16
<211>  29
<212>  DNA
<213>  Artificial

<220>
<223>  specific primer for Peg10

<400>  16
ttaattactc tcctacaact ttccaaatt                                       29


<210>  17
<211>  24
<212>  DNA
<213>  Artificial

<220>
<223>  specific primer for Oct-4

<400>  17
ggttttttag aggatggttg agtg                                            24


<210>  18
<211>  23
<212>  DNA
<213>  Artificial

<220>
<223>  specific primer for Oct-4

<400> 18
tccaacccta ctaacccatc acc 23

## Description

### Technical Field

The present invention relates to a method of producing pluripotent stem cells using testis cells, pluripotent stem cells produced by the method, a method of producing a chimeric embryo, chimeric animal, non-human animal and the like derived from the pluripotent stem cells, a method of producing functional cells such as mesodermal cells from the pluripotent stem cells, a composition for producing pluripotent stem cells derived from testis cells, and the like.

### Background Art

Germ cells are unique in that they have the capacity to contribute genes to the offspring. Although they are highly specialized cells to make gametes for reproduction, several lines of evidences suggest their multipotentiality. For example, teratomas are tumors that nearly always occur in the gonads, and contain many kinds of cells and tissues in various stages of maturation. Furthermore, fetal germ cells are known to give rise to pluripotential cells when cultured under special condition. These embryonic germ (EG) cells have a differentiation property similar to embryonic stem (ES) cells, isolated from inner cell mass. While these observations strongly suggest that the germline lineage may keep the ability to generate pluripotential cells, it has not been possible to establish pluripotent cells from normal postnatal gonads. Because both ES cells and EG cells are collected from prenatal embryos or fetuses, clinical applications thereof to humans pose a major ethical problem, and there has been a demand for the development of a technology for establishing a pluripotent cell from a postnatal individual.

The present inventors have developed a method of the in vitro culture of mouse spermatogonial stem cell, the only stem cell type in the body that can transmit genetic information to offspring (Biol. Reprod., vol.69, p612-616, 2003). When neonatal testis cells were cultured in the presence of glial cell derived neurotrophic factor (GDNF), leukemia inhibitory factor (LIF), epidermal growth factor (EGF), basic fibroblast growth factor (bFGF) and the like, germ cells developed uniquely shaped colonies, and stem cells proliferated over five-months period in a log-scale. Upon transplantation into seminiferous tubules of infertile mice, the cultured cells produced normal sperm and offspring, but no teratoma or somatic cell differentiation was observed, indicating that they are fully committed to the germ cell lineage. This was in contrast to ES cells, which produce teratoma after transferring into seminiferous tubules. Based on these results, we have named these cells, germline stem, or GS, cells to distinguish them from ES or EG cells. Thus, GS cells represent a third method to expand germline cells, but are clearly different from ES/EG cells in their differentiation capacity.

In view of the above-described circumstances, it is an object of the present invention to provide a new method of producing a pluripotent stem cell from a postnatal individual.

### Disclosure of the Invention

The present inventors diligently investigated to accomplish the above-described object and confirmed that when newborn mouse testis cells were cultured under conditions similar to those for GS cell culture, colonies morphologically indistinguishable from ES cell colonies emerge in addition to colonies of GS cells. These ES-like cells grew selectively under ES cell culture conditions. The present inventors found that the ES-like cells have pluripotency as ES cells do since the ES-like cells develop a teratoma when transplanted subcutaneously or otherwise to nude mice, since the ES-like cells are induced to differentiate into diverse functional cells in vitro, and since the ES-like cells exhibit normal embryogenesis and form extremely diverse tissues, including germ cells, when microinjected into blastocysts, and the like, and developed the present invention.

Accordingly, the present invention relates to the following:
(1) A method of producing pluripotent stem cells, which comprises culturing testis cells using a medium containing glial cell derived neurotrophic factor (GDNF) or an equivalent thereto to obtain pluripotent stem cells.
(2) The production method described in (1) above, wherein the medium further contains leukemia inhibitory factor (LIF).
(3) The production method described in (1) or (2) above, wherein the medium further contains at least one of epidermal growth factor (EGF) and basic fibroblast growth factor (bFGF).
(4) The production method described in any one of (1) to (3) above, which comprises culturing testis cells in the presence of feeder cells.
(5) The production method described in (1) above, wherein the testis cells are spermatogonial stem cells.
(6) The production method described in (5) above, wherein the spermatogonial stem cells are GS cells.
(7) The production method described in (1) above, wherein the testis cells are P53-deficient.
(8) The production method described in (1) above, which comprises the following steps:
   (Step 1) culturing testis cells using a medium containing glial cell derived neurotrophic factor (GDNF) or an equivalent thereto to obtain cultured cells;
   (Step 2) culturing the cultured cells obtained in Step 1, using a medium containing leukemia inhibitory factor (LIF) to obtain pluripotent stem cells.
(9) The production method described in (8) above, wherein the medium for Step 1 further contains leukemia inhibitory factor (LIF).
(10) The production method described in (8) or (9) above, wherein the medium for Step 1 further contains at least one of epidermal growth factor (EGF) and basic fibroblast growth factor (bFGF).
(11) The production method described in any one of (8) to (10) above, wherein Step 1 comprises culturing testis cells in the presence of feeder cells.
(12) The production method described in (1) above, which comprises the following steps:
   (Step 1) culturing testis cells using a medium containing glial cell derived neurotrophic factor (GDNF) or an equivalent thereto to obtain GS cells;
   (Step 2) culturing the GS cells obtained in Step 1, using a medium containing glial cell derived neurotrophic factor (GDNF) or an equivalent thereto to obtain pluripotent stem cells.
(13) The production method described in any one of (1) to (12) above, wherein the testis cells are derived from a mammal.
(14) The production method described in (13) above, wherein the mammal is postnatal.
(15) The production method described in (1) above, wherein the pluripotent stem cells are positive for at least any one selected from the group consisting of SSEA-1, Forsman antigen, β1-integrin, α6-integrin, EpCAM, CD9, EE2 and c-kit.
(16) The production method described in (15) above, wherein the pluripotent stem cells are positive for SSEA-1, Forsman antigen, β1-integrin, α6-integrin, EpCAM, CD9, EE2 and c-kit.
(17) A pluripotent stem cell produced by the production method described in any one of (1) to (16) above.
(18) A pluripotent stem cell derived from a testis cell, which is positive for at least any one selected from the group consisting of SSEA-1, Forsman antigen, β1-integrin, α6-integrin, EpCAM, CD9, EE2 and c-kit.
(19) The pluripotent stem cell described in (18) above, which is positive for SSEA-1, Forsman antigen, β1-integrin, α6-integrin, EpCAM, CD9, EE2 and c-kit.
(20) A method of producing a chimeric embryo, which comprises the following steps:
   (Step 1) culturing testis cells using a medium containing glial cell derived neurotrophic factor (GDNF) or an equivalent thereto to obtain pluripotent stem cells;
   (Step 2) introducing the pluripotent stem cells into a host embryo to obtain a chimeric embryo.
(21) A method of producing a chimeric animal (excluding humans), which comprises the following steps:
   (Step 1) culturing testis cells using a medium containing glial cell derived neurotrophic factor (GDNF) or an equivalent thereto to obtain pluripotent stem cells;
   (Step 2) introducing the pluripotent stem cells into a host embryo to obtain a chimeric embryo;
   (Step 3) transferring the chimeric embryo to the uterus or oviduct of a host animal to obtain a chimeric animal (excluding humans).
(22) A method of producing a non-human animal derived from pluripotent stem cells, which comprises the following steps:
   (Step 1) culturing testis cells using a medium containing glial cell derived neurotrophic factor (GDNF) or an equivalent thereto to obtain pluripotent stem cells;
   (Step 2) introducing the pluripotent stem cells into a host embryo to obtain a chimeric embryo;
   (Step 3) transferring the chimeric embryo to the uterus of a host animal to obtain a chimeric animal (excluding humans); (Step 4) mating the chimeric animal to obtain a non-human animal derived from the pluripotent stem cells.
(23) A method of producing a tetraploid chimeric embryo, which comprises the following steps:
   (Step 1) culturing testis cells using a medium containing glial cell derived neurotrophic factor (GDNF) or an equivalent thereto to obtain pluripotent stem cells;
   (Step 2) introducing the pluripotent stem cells into a tetraploid embryo to obtain a tetraploid chimeric embryo.
(24) A method of producing a non-human animal derived from pluripotent stem cells, which comprises the following steps:
   (Step 1) culturing testis cells using a medium containing glial cell derived neurotrophic factor (GDNF) or an equivalent thereto to obtain pluripotent stem cells;
   (Step 2) introducing the pluripotent stem cell into a tetraploid embryo to obtain a tetraploid chimeric embryo;
   (Step 3) transferring the tetraploid chimeric embryo to the uterus or oviduct of a host animal to obtain a non-human animal derived from the pluripotent stem cells.
(25) A method of producing functional cells, which comprises the following steps:
   (Step 1) culturing testis cells using a medium containing glial cell derived neurotrophic factor (GDNF) or an equivalent , thereto to obtain pluripotent stem cells;
   (Step 2) culturing the pluripotent stem cells under functional cell differentiation conditions to obtain functional cells.
(26) The production method described in (25) above, wherein the functional cells are mesodermal cells.
(27) The production method described in (26) above, wherein the mesodermal cells are any one selected from the group consisting of blood cell lineage cells, vascular lineage cells and myocardial cells.
(28) The production method described in (25) above, wherein the functional cells are ectodermal cells.
(29) The production method described in (28) above, wherein the ectodermal cells are neuronal lineage cells.
(30) The method described in (29) above, wherein the neuronal lineage cells are any one selected from the group consisting of neurons, glial cells, oligodendrocytes and astrocytes.
(31) The production method described in (25) above, wherein the functional cells are endodermal cells.
(32) A composition for producing pluripotent stem cells derived from a testis cell, which contains glial cell derived neurotrophic factor (GDNF) or an equivalent thereto.
(33) The composition described in (32) above, which further contains leukemia inhibitory factor (LIF).
(34) The composition described in (32) or (33) above, which further contains at least one of epidermal growth factor (EGF) and basic fibroblast growth factor (bFGF).

Using the production method of the present invention, it is possible to produce pluripotent stem cells such as ES cells and EG cells, which have conventionally been only obtainable from a prenatal individual (a fertilized egg, an embryo and the like), from a postnatal individual. Using the pluripotent stem cells, it is possible to construct diverse tissues having histocompatibility for autotransplantation, and the pluripotent stem cells are useful in medical fields such as regeneration medicine and gene therapy. The pluripotent stem cells are also useful in the field of biotechnology because they can be used to prepare a genetically modified animal such as a transgenic animal or a knockout animal.

### Brief Description of the Drawings

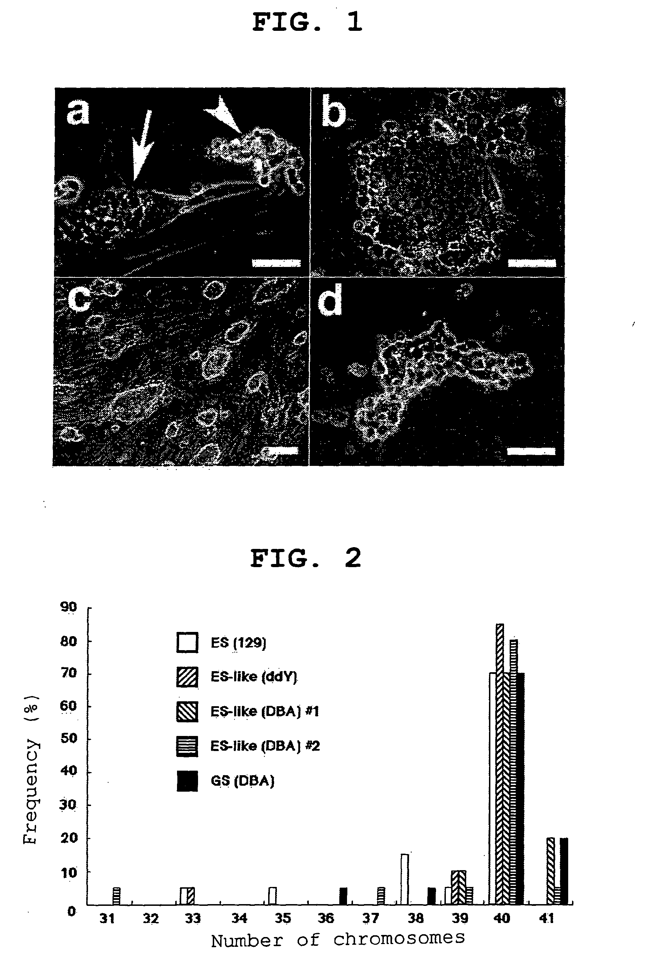

FIG. 1 presents photographs showing the morphologies of colonies of GS cells and pluripotent stem cells obtained by the production method of the present invention. In each of the panels "a" to "d", the bar in the lower right indicates 50 µm. "a" is a photograph showing a state wherein colonies of GS cells (white arrowhead) and colonies of the pluripotent stem cells (white arrow) are co-present in the initial stage of cultivation. "b" is a photograph showing the morphology of colonies of the pluripotent stem cells in the initial stage of cultivation. The pluripotent stem cells are more densely packed. "c" is a photograph showing the morphology of colonies of the completely established pluripotent stem cells. The morphology of the colonies is completely like ES cell colonies. "d" is a photograph showing the morphology of a typical colony of GS cells.
FIG. 2 shows distribution of metaphase spreads with different chromosome numbers. At least 20 cells were counted.
These are results from ES cells [ES(129)], ddY-mouse-derived ES-like cells [ES-like (ddY)], DBA/2-mouse-derived ES-like cells [ES-like (DBA)] and DBA/2-mouse-derived GS cells [GS (DBA)]. The ordinate indicates frequency (%); the abscissa indicates the number of chromosomes.

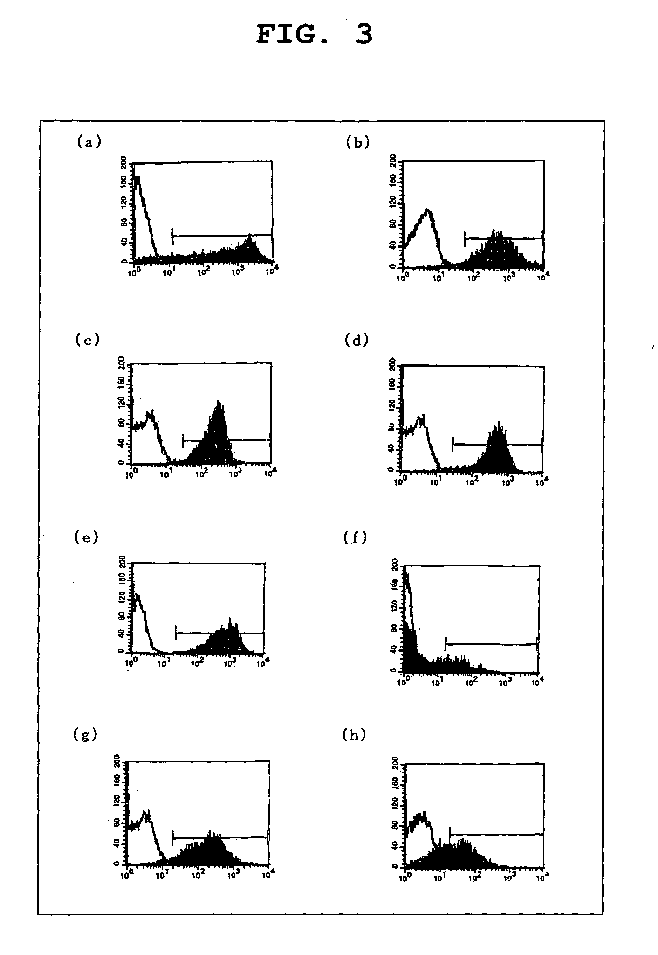

FIG. 3 presents histograms showing the expression of cell surface markers for pluripotent stem cells obtained by the production method of the present invention. There is shown the expression of (a) SSEA-1, (b) β1-integrin, (c) α6-integrin, (d) EpCAM, (e) CD9, (f) Forsman antigen, (g) EE2, and (h) c-kit. The ordinate indicates the number of cells; the abscissa indicates the expression of each cell surface marker as relative intensity of fluorescence. The white columns show histograms in cases where the cells were stained without using a primary antibody (negative control); the black columns show histograms in cases where the cells were stained using a primary antibody. The ratio (%) of cells in the gate to the total cell number is (a) 85.14%, (b) 93.72%, (c) 97.98%, (d) 96.36%, (e) 99.11%, (f) 25.38%, (g) 92.29%, and (h) 57.88%, respectively.

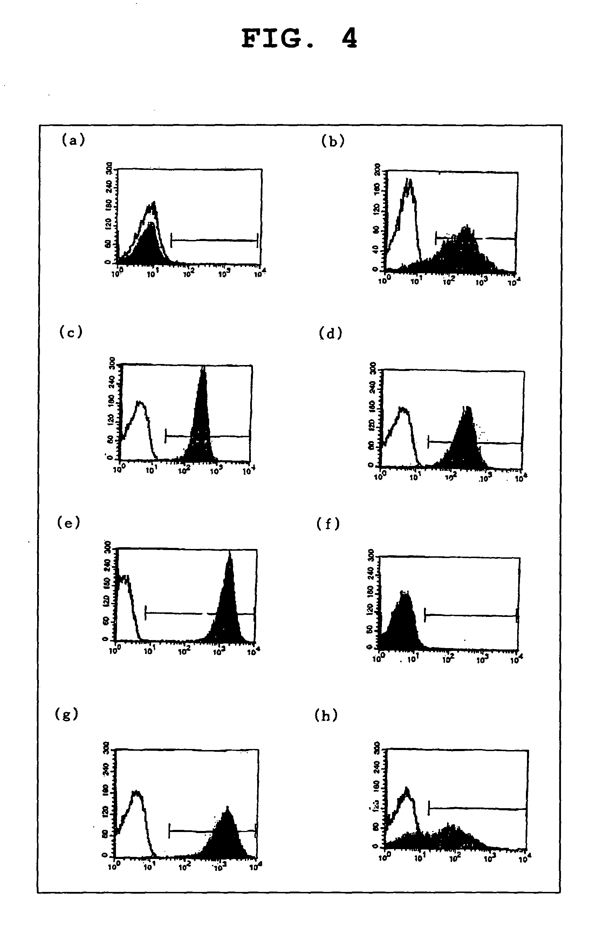

FIG. 4 presents histograms showing the expression of cell surface markers for GS cells. There is shown the expression of (a) SSEA-1, (b) β1-integrin, (c) α6-integrin, (d) EpCAM, (e) CD9, (f) Forsman antigen, (g) EE2, and (h) c-kit. The ordinate indicates the number of cells; the abscissa indicates the expression of each cell surface marker as relative intensity of fluorescence. The white columns show histograms in cases where the cells were stained without using a primary antibody (negative control); the black columns show histograms in cases where the cells were stained using a primary antibody. The ratio (%) of cells in the gate to the total cell number is (a) 0.67%, (b) 84.83%, (c) 99.70%, (d) 99.20%, (e) 99.11%, (f) 1.72%, (g) 92.78%, and (h) 64.14%, respectively.

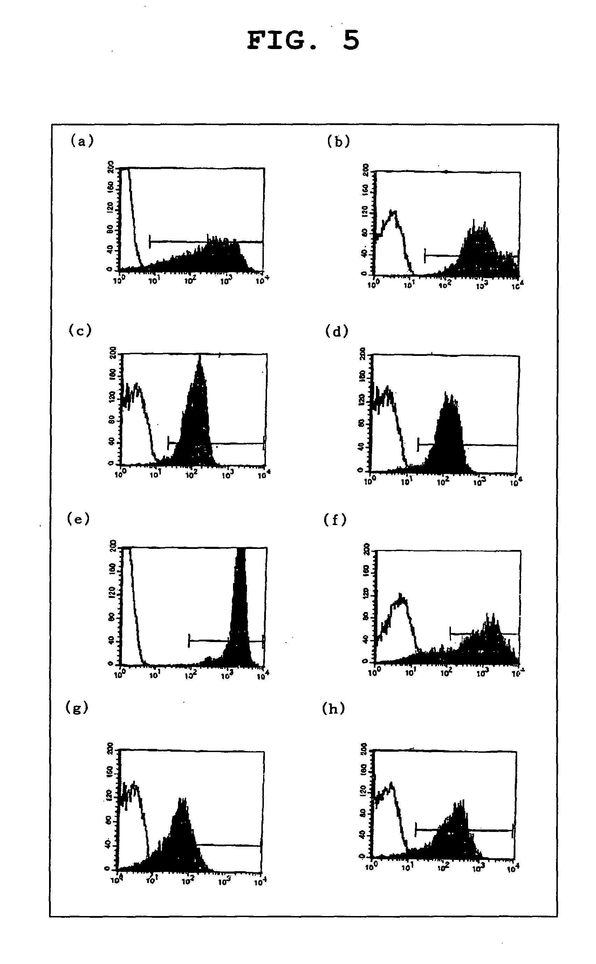

FIG. 5 presents histograms showing the expression of cell surface markers for ES cells. There is shown the expression of (a) SSEA-1, (b) β1-integrin, (c) α6-integrin, (d) EpCAM, (e) CD9, (f) Forsman antigen, (g) EE2, and (h) c-kit. The ordinate indicates the number of cells; the abscissa indicates the expression of each cell surface marker as relative intensity of fluorescence. The white columns show histograms in cases where the cells were stained without using a primary antibody (negative control); the black columns show histograms in cases where the cells were stained using a primary antibody. The ratio (%) of cells in the gate to the total cell number is (a) 96.46%, (b) 99.69%, (c) 97.23%, (d) 96.10%, (e) 99.68%, (f) 79.11%, (g) 81.78%, and (h) 93.90%, respectively.

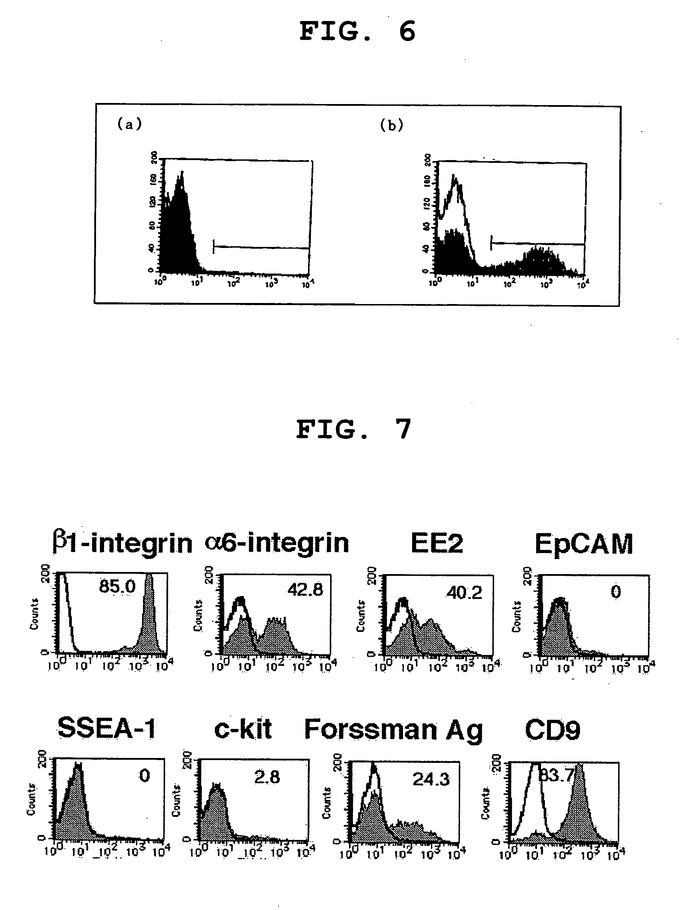

FIG. 6 presents histograms showing the expression of cell surface markers for testis cells before the start of , cultivation. There is shown the expression of (a) SSEA-1 and (b) Forsman antigen. The ordinate indicates the number of cells; the abscissa indicates the relative intensity of fluorescence. The white columns show histograms in cases where the cells were stained without using a primary antibody (negative control); the black columns show histograms in cases where the cells were stained using a primary antibody. The ratio (%) of cells in the gate to the total cell number is (a) 0.92% and (b) 43.02%, respectively.

FIG. 7 presents histograms showing the expression of cell surface markers for testis cells before the start of cultivation. The ordinate indicates the number of cells; the abscissa indicates the relative intensity of fluorescence. The white columns show histograms in cases where the cells were stained without using a primary antibody (negative control); the gray columns show histograms in cases where the cells were stained using a primary antibody. The ratio (%) of positive cells to the total cell number is shown in each histogram.

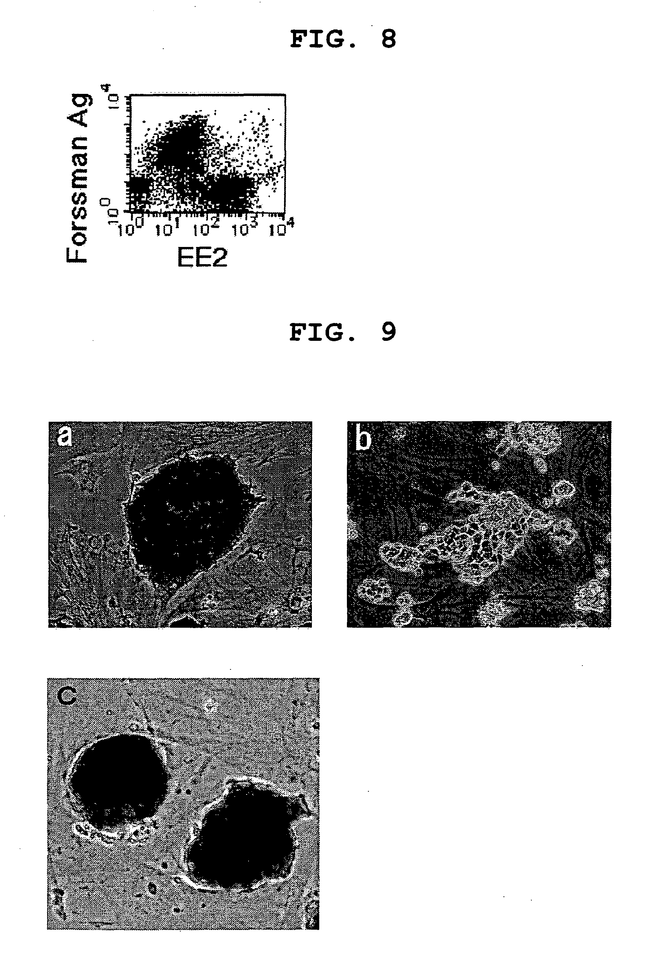

FIG. 8 shows the results of double immunostaining of neonatal testis cells by anti-EE2 and anti-Forssman antigen antibodies.

FIG. 9 shows the results of alkaline phosphatase staining. There are shown (a) a colony of pluripotent stem cells obtained by the production method of the present invention, (b) a colony of GS cells, and (c) colonies of ES cells, respectively.

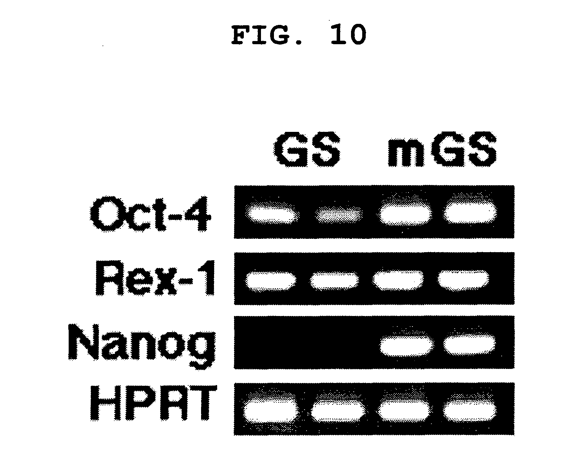

FIG. 10 shows the results of RT-PCR analysis. The expression of OCT-4, Rex-1, Nanog and HPRT in GS cells (GS) and pluripotent stem cells obtained by the production method of the present invention (mGS) is shown.

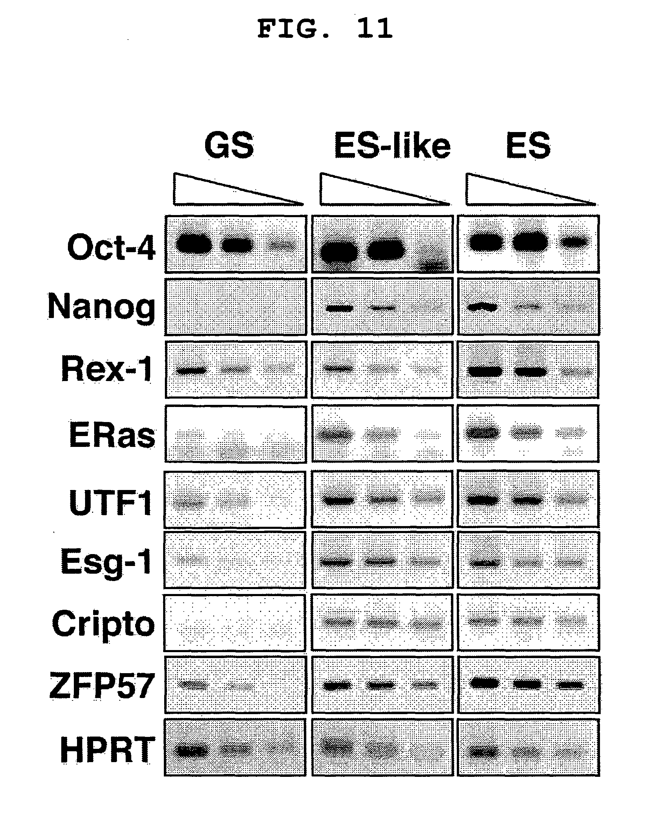

FIG. 11 shows the results of RT-PCR analysis. Three-fold serial dilutions of cDNA from GS, ES-like and ES cells were amplified with specific primers.

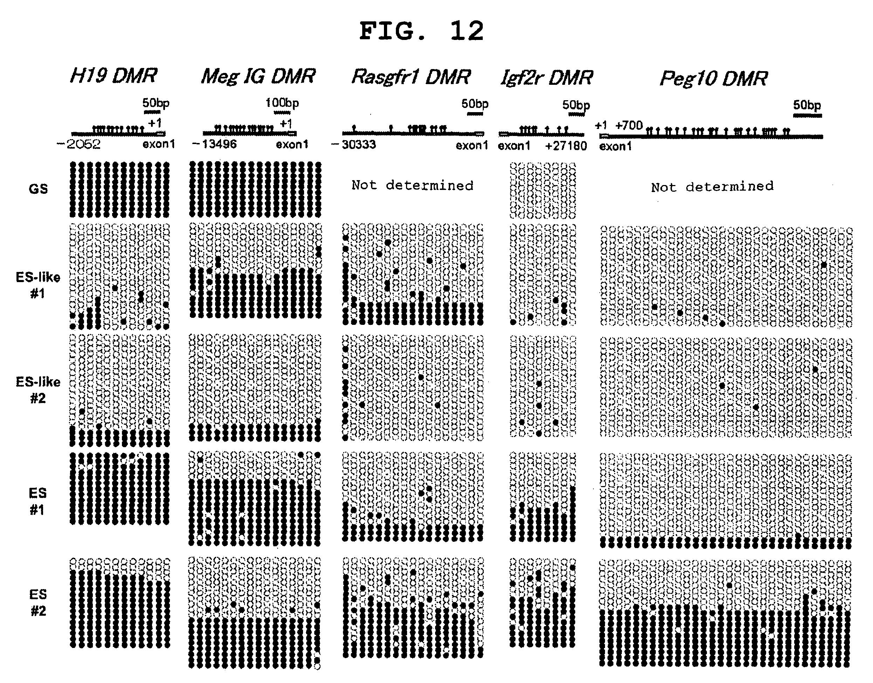

FIG. 12 shows the results of Analysis of Imprinting in ES-Like Cells. DMR methylation of H19, Meg3 IG, Rasgrf1, Igf2r, and Peg10 regions are shown. DNA methylation was analyzed by bisulfate genomic sequencing. Black ovals indicate methylated cytosine-guanine sites (CpGs), and white ovals indicate unmethylated CpGs.

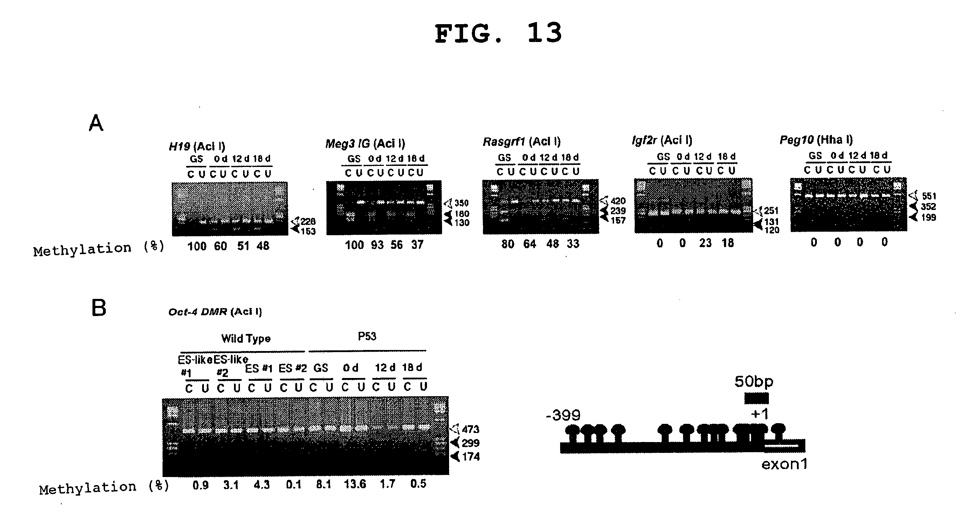

FIG. 13 shows the results of Analysis of Imprinting in ES-Like Cells.
(A) COBRA of GS and ES-like cells from p53 knockout mice. The day when ES-like colonies were found was designated day 0, and cells were collected at the indicated time. In this culture, only ES-like cells were found by day 12. Each numerical figure in the bottom of each panel shows the ratio (%) of methylation.
(B) COBRA of the upstream region of the Oct-4 gene in ES-like cells from a wild type mouse (Wild Type) and a P53 knockout mouse (P53). Each numerical figure in the bottom of the left panel shows the ratio (%) of methylation. The right panel is a schematic diagram of the upstream region of the Oct-4 gene. Open arrowheads indicate the size of unmethylated DNA. Closed arrowheads indicate the size of methylated DNA. Enzymes used to cleave each locus are indicated in parentheses. U, uncleaved; C, cleaved.

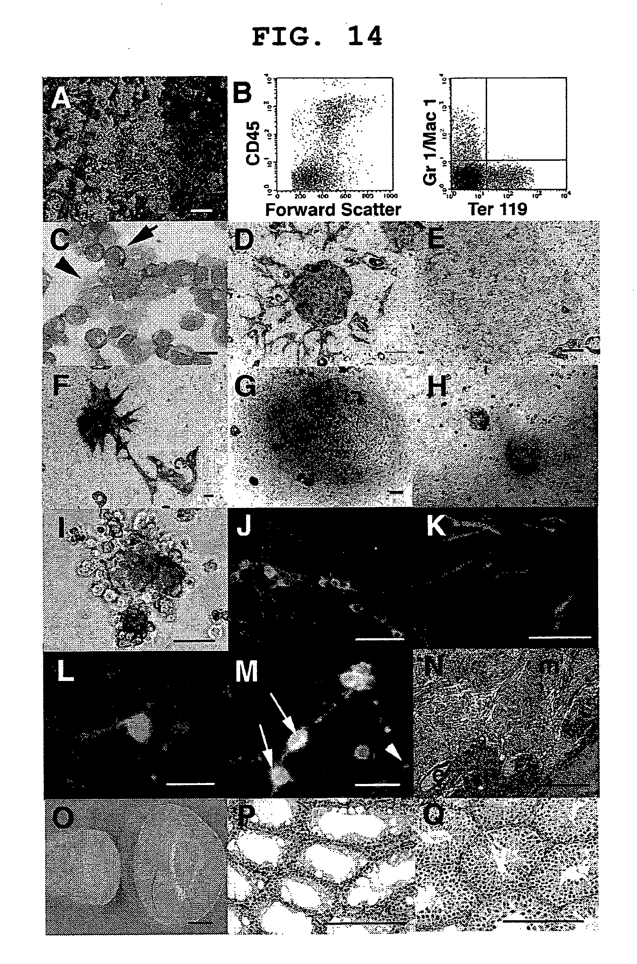

FIG. 14 shows In Vitro and In Vivo Differentiation of ES-Like Cells. (A-H) Differentiation on OP9 cells. (A) Cobblestone structure (hematocyte) on day 8. (B) CD45-positive hematopoietic cell development on day 7 after coculture (left). In this cell population, Gr1-positive granulocytes, Mac1-positive macrophages, or Ter119-positive erythrocytes were found (right). (C) May-Giemsa staining of harvested cells. Myeloid progenitor (arrowhead) and erythroblast (arrow) were observed. (D and E) show vascular cell (endothelial cell and the like) differentiation. Flk-1-positive cells were sorted on day 4 after coculture, and CD31-positive (D) or VE-cadherin-positive (E) vascular cells appeared at 6 days after cell sorting. (F-H) Heart muscle differentiation. The Flk-1-positive cells were differentiated into MF20-positive (F) or cTn-I-positive (G) heart muscle at 6 days after sorting. (H), ANP-positive (blue) atrial muscle and MLC2v-positive (brown) ventricular muscle. (I) Erythroid cells that developed from embryoid body in methylcellulose at 8 days after culture. Note the red color of the cells. The cell shows a red color. (J-M) Neuronal cell differentiation on gelatin-coated plates. Tuj-positive neurons (J) on day 5, GFAP-positive astrocytes (K) and MBP-positive oligodendrocytes (L) on day 7 after induction. TH and Tuj-double positive dopaminergic neurons (arrow) appeared among Tuj-positive neurons (arrowhead) (M). (N) Section of a teratoma under the skin. The tumors contained a variety of differentiated cell types, including muscle (m), neural (n), and epithelial (e) tissues. (O-Q) Spermatogenesis from p53 knockout GS cells. (O) A macroscopic comparison of untransplanted (left) and transplanted (right) recipient testes. Note the increased size of the transplanted testis. (P and Q) Histological appearance of the untransplanted (P) and transplanted (Q) W testes. Note the normal appearance of spermatogenesis (Q). Color staining: Cy3, red. Color staining: Cy3, red (J-M); Alexa Fluor 488, green (M). Scale bar, 50 µm (A, D-I, J, K, and M), 20 µm (C and L), 200 µm (N, P, and Q), 1 mm (O).

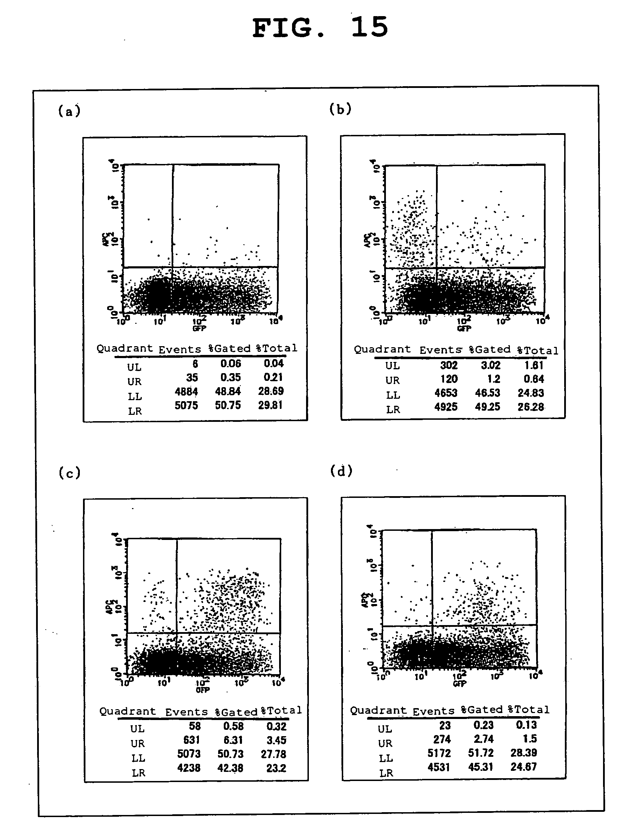

FIG. 15 shows the results of flowcytometry analysis. There are shown the analytical results for (a) negative control, (b) Ter119, (c) CD45, and (d) Mac1/Gr1 (stained with Mix). In (a) to (d), in each upper dot plot, the ordinate indicates the expression of each cell surface marker; the abscissa indicates the expression of EGFP as relative intensity of fluorescence, respectively. In (a) to (d), each table on the bottom shows the number of plots (events), the ratio (% gate) to the number of all surviving cells (% gate), and the ratio (% entire) to the number of all cells in each of the four separate gates (upper left, upper right, lower left, lower right).

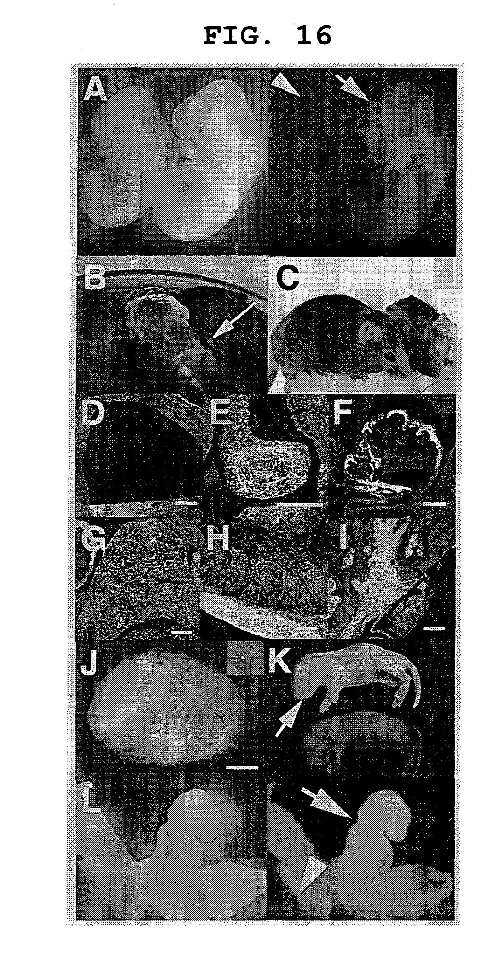

FIG. 16 shows preparation of chimeric animals. (A) A 12.5 dpc chimeric embryo (arrow) showing fluorescence under UV light. No fluorescence was observed in a control embryo (arrowhead). The left drawing shows observation under visible light. (B) A newborn chimeric animal (arrow) showing fluorescence. (C) Mature chimeric animals. Note the donor cell-derived coat color (cinnamon). (D-I) Parasagittal section of a 12.5 dpc chimeric embryo. Fluorescence was observed in the brain (D), intestine (E), heart (F), liver (G), lower spinal cord (H), and placenta (I). (J) A testis from a chimeric mouse showing fluorescence. EGFP expression was observed in some germ cells in the testis cell suspension (inset). (K) Offspring derived from a chimera. One of the offspring showed fluorescence, confirming the donor origin (arrow). (L) A 10.5 dpc embryo (arrow) and yolk sac produced from an aggregation of ES-like cells with tetraploid embryo showing fluorescence. No fluorescence was observed in the placenta (arrowhead). Counterstained with propidium iodide (PI) (D-I). Color staining: EGFP, green (A, B, and D-L); PI, red (D-I). Scale bar, 100 µm (D-I), 1 mm (J).

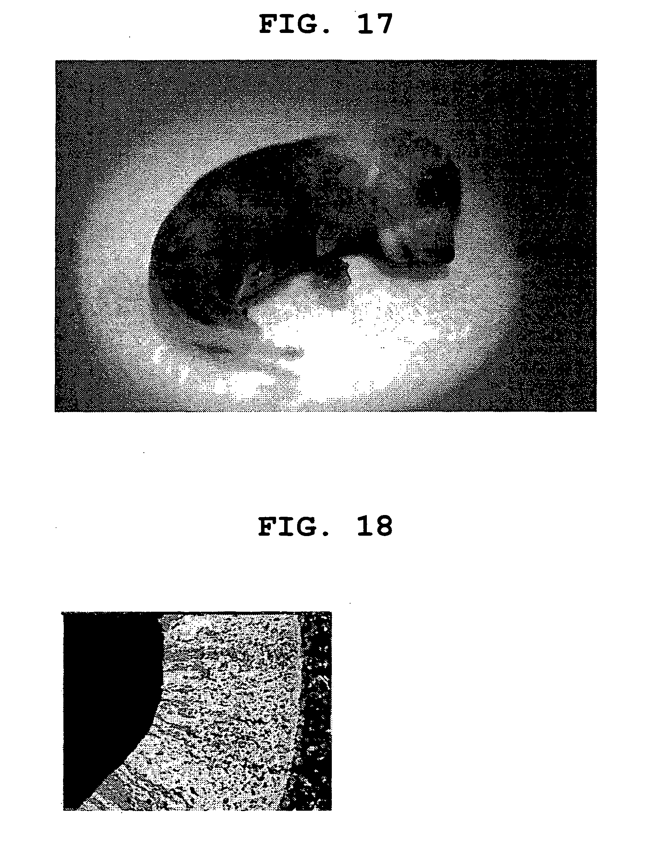

FIG. 17 shows the results of an examination of a newborn chimeric mouse pup under UV light.

FIG. 18 shows the results of a histological examination of a frozen section of a chimeric mouse fetus using a fluorescent microscope [lower portion of the spinal cord (neural tube)].

### Detailed Description of the Invention

The method of the present invention of producing pluripotent stem cells comprises culturing testis cells using a medium containing glial cell derived neurotrophic factor (GDNF) or an equivalent thereto to obtain (for example, isolate, separate, sorte, purificate and the like) pluripotent stem cells [herein sometimes referred to as ES-like cells or multipotent germline stem cells (mGS cells)].

A pluripotent stem cell refers to a cell that can be cultured in vitro, is capable of growing over a long period, has a self-replicating potential, and has the capability of differentiating into all cells constituting a living organism or precursor cells thereof.

Testis cells include all cells constituting the testis, for example, spermatogonial stem cells, spermatogonia, spermatids, spermatogonia, primary spermatocytes, secondary spermatocytes, spermatozoa, Leydig cells, Sertoli cells, interstitial cells, gonocytes, germ cells and the like can be mentioned.

A spermatogonial stem cell refers to a germline cell having the capability of self-regenerating and differentiating into a spermatozoon or a precursor cell thereof (for example, spermatogonium, spermatid, spermatogonium, primary spermatocyte, secondary spermatocyte and the like) (capability as a spermatogonial stem cell). As examples of the spermatogonial stem cell, primordial germ cell, gonocyte, spermatogonium which is a stem cell (a cell having the capability as spermatogonial stem cells out of spermatogonia), germline stem cell (GS cell) and the like can be mentioned. In the present invention, the spermatogonial stem cell is preferably a gonocyte, spermatogonium which is a stem cell, or GS cell.

A GS cell refers to a spermatogonial stem cell grown dependently on a GDNF receptor agonist compound (GDNF or an equivalent thereto) in vitro, for example, a spermatogonial stem cell grown by the method described in Biol. Reprod., vol.69, p612-616, 2003.

Testis cells can be prepared from the testis by a method known per se. For example, the testis is extirpated, and the extirpated testis is digested with a lytic enzyme such as collagenase, trypsin, and DNase to disperse testis cells (see, for example, Biol. Reprod., vol.69, p612-616, 2003 and the like). The testis cells are washed with culture medium and the like and used to produce the pluripotent stem cells of the present invention. ,

The testis cells may be cultured before being used to produce the pluripotent stem cells of the present invention. Culture conditions are not subject to limitation; for example, as described in Biol. Reprod., vol.69, p612-616, 2003, by culturing testis cells obtained by the above-described enzyme treatment in the presence of glial cell derived neurotrophic factor (GDNF), leukemia inhibitory factor (LIF) and the like, spermatogonial stem cells may be grown to obtain GS cells, which may be used.

The testis cells may also be those obtained by concentrating a fraction of high capability of producing pluripotent stem cells, before being used to produce the pluripotent stem cell of the present invention. As examples of the fraction, spermatogonial stem cells, spermatogonia, gonocytes, germ cells and the like can be mentioned.

As examples of the method of concentration, a method using an antibody that recognizes a cell surface antigen specifically expressed in the cells of the fraction and using a cell sorter, antibody magnetic microbeads and the like, and the like can be mentioned. For example, spermatogonial stem cells can be concentrated with a cell surface antigen such as α6-integrin, c-kit, or CD9 as the indicator (see, for example, Proc Natl Acad Sci USA, 97, 8346-8351, 2001; Biol. Reprod., vol.70, p70-75, 2004 and the like). Alternatively, it is also possible to concentrate spermatogonial stem cells using a dye of Hoechst and the like (see Development, 131, 479-487, 2004 and the like).

The testis cell used in the present invention is not subject to limitation, as long as it is derived from an animal. The animal is not subject to limitation, as long as pluripotent stem cells can be produced by the method of the present invention, and the animal may be any of a vertebrate and an invertebrate, and is preferably a vertebrate.

As examples of the vertebrate, a mammal, bird, fish, amphibian and reptile can be mentioned. Examples of the mammal include, but are not limited to, laboratory animals such as mice, rats, hamsters, guinea pigs and other rodents, and rabbits, domestic animals such as pigs, bovines, goat, horses, and sheep, pet animals such as dogs and cats, and primates such as humans, monkeys, orangutans, and chimpanzees. As the bird, chicken, partridges, ducks, geese, turkeys, ostriches, emus, ostriches, guinea fowls, pigeons and the like can be mentioned.

The vertebrate is preferably a mammal.

Although the mammal may be prenatal or postnatal, as long as pluripotent stem cells can be produced by the method of the present invention, it is preferably postnatal.

When a prenatal fetus is used, the developmental stage of the fetus is not subject to limitation, as long as pluripotent stem cells can be produced by the method of the present invention; for example, a developmental stage on and after the formation of the male genital ridge can be mentioned. In mice, for example, a developmental stage after 12.5 dpc, for example, on and after 13.0 dpc, preferably on and after 13.5 dpc, more preferably on and after 14.5 dpc, still more preferably on and after 16.5 dpc, can be mentioned.

When a postnatal animal is used, the age of the animal is not subject to limitation, as long as pluripotent stem cells can be produced by the method of the present invention; although the animal may be any of a neonate, infant, adult, and aged animal, it is preferable, from the viewpoint of production efficiency, to use a younger animal because younger animals have higher frequencies of stem cells (spermatogonial stem cells and the like) contained in the testis and the like and for other reasons. That is, the animal used is preferably a neonate or infant, more preferably a neonate. Here, an adult refers to an individual having reached sexual maturation (for example, 4 weeks or more of age for mice), an infant refers to an individual not having reached sexual maturation but exhibiting spermatogenesis (for example, 5 days to 4 weeks of age for mice), and a neonate refers to an individual prior to the start of spermatogenesis (for example, 0 to 4 days of age for mice).

When a mouse is used as the postnatal animal, the age of the mouse is not subject to limitation, as long as pluripotent stem cells can be produced by the method of the present invention; for example, the mouse is 0 to 8 weeks of age, preferably 0 to 3 weeks of age, more preferably 0 to 8 days of age, most preferably 0 to 2 days of age. 0 day (week) of age means the day of birth.

P53-deficient testis cells may be used as the testis cells used in the present invention. By using P53-deficient testis cells in the production method of the present invention, it is sometimes possible to obtain pluripotent stem cells at extremely higher efficiency compared with the use of wild type testis cells. In particular, in cases where adult-derived cells or GS cells are used as the testis cells and other cases, the utilization of P53-deficient testis cells is advantageous.

"P53-deficient" refers to a state wherein the P53 gene is functionally deficient, that is, a state wherein the P53 gene cannot fully exhibit the normal function essentially possessed thereby; a state wherein the P53 gene is not expressed at all, a state wherein the amount of the P53 gene expressed has been decreased to the extent that the gene cannot fully exhibit the normal function essentially possessed thereby, a state wherein the P53 gene product has completely lost the function thereof, or a state wherein the function of the P53 gene has been decreased to the extent that the gene cannot fully exhibit the normal function essentially possessed thereby, can be mentioned.

As examples of P53-deficient testis cells, a P53 knockout homozygote or a P53 knockout heterozygote, preferably a P53 knockout homozygote, can be mentioned. P53-deficient testis cells can be obtained by, for example, recovering testis cells of a P53-deficient animal (P53 knockout animals and the like). Alternatively, it is also possible to obtain P53-deficient , testis cells by introducing a targeting vector for the P53 gene into testis cells, and deleting the P53 gene by homologous recombination.

In another mode of embodiment, P53-deficient testis cells can be produced by introducing a substance that suppresses the expression or function of the P53 gene [for example, antisense nucleic acids, RNAi inducing nucleic acids (siRNA, stRNA, miRNA and the like) and the like] into cells. Introduction of the substance that suppresses the expression or function of P53 into testis cells can be performed by a method known per se; for example, when the substance that suppresses the expression or function of the P53 gene is a nucleic acid molecule or an expression vector harboring the same, the calcium phosphate method, lipofection method/liposome method, electroporation method and the like can be used.

The term "equivalent to glial cell-derived neurotrophic factor (GDNF)" as used herein encompasses, though not particularly limited as long as the production of pluripotent stem cells can be achieved when subjected to the method of the present invention, GDNF-like compounds such as neurturin, persephin, and artemin, and other compounds exhibiting an action similar to that of glial cell-derived neurotrophic factor (GDNF) or a GDNF-like compound on a GDNF receptor(s) or an co-receptor(s) (for example, antibodies that specifically recognize a GDNF receptor(s) or an co-receptor(s), agonistic compounds to a GDNF receptor(s) or an co-receptor(s), and the like). As such, the receptor(s) or co-receptor(s) include Ret tyrosine kinase and the GDNF-family receptor α:s, respectively.

A GDNF-like compound means a compound that is structurally similar to glial cell-derived neurotrophic factor (GDNF), or that acts like glial cell-derived neurotrophic factor (GDNF) on a receptor or co-receptor thereof, or that capable of producing pluripotent stem cells when subjected to the method of the present invention. As the GDNF-like compound, neurturin, persephin, artemin and the like, in particular, can be mentioned.

Glial cell-derived neurotrophic factor (GDNF) and GDNF-like compounds are structurally similar to each other; cRet receptor tyrosine kinase acts as a common signal transmission receptor shared by glial cell-derived neurotrophic factor (GDNF), neurturin, persephin, and artemin.

A compound that acts like glial cell-derived neurotrophic factor (GDNF)" means a compound that acts in the same manner as glial cell-derived neurotrophic factor (GDNF) on a receptor that transmits the signal of glial cell-derived neurotrophic factor (GDNF) or a co-receptor thereof.

"A GDNF receptor" means a substance that binds to glial cell-derived neurotrophic factor (GDNF) or a GDNF-like compound, i.e., a compound capable of transmitting the signal of glial cell-derived neurotrophic factor (GDNF) or a GDNF-like compound. As the "GDNF receptor", cRet receptor tyrosine kinase, which is a receptor that mediates a signal for glial cell-derived neurotrophic factor (GDNF) or GDNF-like compound, in particular, can be mentioned.

"A GDNF co-receptor" means a receptor that does not transmit the signal of glial cell-derived neurotrophic factor (GDNF) or a GDNF-like compound but activates a receptor that transmits the signal of glial cell-derived neurotrophic factor (GDNF) or a GDNF-like compound. These compounds, in particular, are receptors whose members are called the GDNF family receptor α:s (GFRα). These are also associated with signaling receptor complexes for GDNF, persephin, artemin, and neurturin. As receptors of the family, 4 members (GFRα 1 to 4) (Jing, S., et al., Cell, 85, 9-10 (1996); Jing, S. Q., et al., J. Biol. Chem., 272, 33111-33117 (1997); Trean or, J. J., et al., Nature, 382, 80-83 (1996); Subanto, P., et al., Human Molecular Genetics, 6, 1267-1273 (1997)) are already known. These are capable of independently transmitting signals, all of which are essential to ligand binding and cRet activation.

In the production method of the present invention, the concentration of the glial cell derived neurotrophic factor (GDNF) or an equivalent thereto contained in the medium is not subject to limitation, as long as pluripotent stem cells can be produced by the method of the present invention, and it is generally 0.05 ng/ml to 100 mg/ml, for example, 0.5 ng/ml to 100 µg/ml, preferably 0.5 ng/ml to 10 µg/ml, more preferably 0.5 ng/ml to 1 µg/ml, still more preferably 0.5 to 200 ng/ml, still yet more preferably 0.5 to 50 ng/ml, most preferably 2 to 20 ng/ml.

The medium used in the production method of the present invention preferably further contains leukemia inhibitory factor (LIF).

In the production method of the present invention, when leukemia inhibitory factor (LIF) is contained in the medium, the concentration thereof is not subject to limitation, as long as pluripotent stem cells can be produced by the method of the present invention, and it is generally 10 to 10⁶ units/ml, for example, 10 to 10⁵ units/ml, preferably 10² to 10⁴units/ml, more preferably 3x10² to 5x10³ units/ml.

The medium used in the production method of the present invention preferably further contains at least one of epidermal growth factor (EGF) and basic fibroblast growth factor (bFGF), more preferably both.

In the production method of the present invention, when epidermal growth factor (EGF) is contained in the medium, the concentration thereof is not subject to limitation, as long as pluripotent stem cells can be produced by the method of the present invention, and it is generally 0.05 ng/ml to 100 mg/ml, for example, 0.5 ng/ml to 100 µg/ml, preferably 0.5 ng/ml to 10 µg/ml, more preferably 0.5 ng/ml to 1 µg/ml, still more preferably 0.5 to 200 ng/ml, still yet more preferably 0.5 to 50 ng/ml, most preferably 2 to 30 ng/ml.

In the production method of the present invention, when basic fibroblast growth factor (bFGF) is contained in the medium, the concentration thereof is not subject to limitation, as long as pluripotent stem cells can be produced by the method of the present invention, and it is generally 0.05 ng/ml to 100 mg/ml, for example, 0.5 ng/ml to 100 µg/ml, preferably 0.5 ng/ml to 10 µg/ml, more preferably 0.5 ng/ml to 1 µg/ml, still more preferably 0.5 to 200 ng/ml, still yet more preferably 0.5 to 50 ng/ml, most preferably 2 to 20 ng/ml.

The cytokine that can be contained in the medium in the present invention [glial cell derived neurotrophic factor (GDNF), leukemia inhibitory factor (LIF), epidermal growth factor (EGF) and basic fibroblast growth factor (bFGF) and the like] may be any one derived from an animal, preferably from the above-described mammal, without limitation, as long as pluripotent stem cells can be produced by the method of the present invention.

As examples of the glial cell derived neurotrophic factor (GDNF), glial cell derived neurotrophic factors (GDNFs) of humans and rats (WO93/06116 pamphlet), mice (see, for example, Gene 203, 2, 149-157, 1997) and the like can be mentioned.

As examples of the leukemia inhibitory factor (LIF), leukemia inhibitory factors (LIFs) of humans (JP-A-1-502985), mice (JP-A-1-502985), sheep (JP-A-4-502554), pigs (JP-A-4-502554) , bovines (JP-A-8-154681) and the like can be mentioned.

As examples of the epidermal growth factor (EGF), epidermal growth factors (EGFs) of mice (see, for example, Nature, 257, 325-327, 1975), humans (see, for example, Proc Natl Acad Sci USA, 88, 415, 1991) and the like can be mentioned.

As examples of the basic fibroblast growth factor (bFGF), human bFGF (see, for example, Endocrine Rev., 8, 95, 1987), bovine bFGF (see, for example, Proc. Natl. Acad. Sci. USA, 81, 6963, 1984), mouse bFGF (see, for example, Dev. Biol., 138, 454-463, 1990), rat bFGF (see, for example, Biochem. Biophys. Res. Commun., 157, 256-263, 1988) and the like can be mentioned. ,

Also, the cytokine comprises a purified naturally occurring, synthetic or recombinant protein, a mutant protein (including insertion, substitution and deletion mutants), a fragment, and a chemically modified derivative thereof, as long as pluripotent stem cells can be acquired when the cytokine is used in the method of the present invention of producing pluripotent stem cells. The cytokine also comprises a protein substantially homologous to the wild type amino acid sequence of each of the above-described cytokines.

The number of amino acids inserted, substituted or deleted in the mutant protein is generally 1 to 20, preferably 1 to 10, more preferably 1 to 5, most preferably 1 or 2.

"Substantially homologous" means that the degree of homology to the wild type amino acid sequence is preferably 70% or more, more preferably 80% or more, still more preferably 90% or more, most preferably 95% or more. The ratio of homology (%) is calculated as the ratio (%) of amino acid residues present in the lesser of two sequences in alignment that are identical amino acids in the sequence to be compared with, provided that four gaps can be introduced into a length of 100 amino acids to help sequence alignment, as described in the Atlas of Protein Sequence and Structure v.5, p.124, National Biochemical Research Foundation, Washington, D.C. (1972). Also, an optionally chosen protein that can be isolated on the basis of the cross-reactivity to the antibody against each of the above-described cytokines having the wild type amino acid sequence, and a protein encoded by a gene that isolated by hybridization with the gene that encodes the wild type amino acid sequence of each of the above-described cytokines or a gene segment thereof under stringent conditions, are included as the substantially homologous protein.

As examples of the above-described stringent conditions, the hybridization conditions described by Sambrook, J. et al. in "Expression of cloned genes in E. coli" [Molecular Cloning: A laboratory manual (1989), Cold Spring Harbor Laboratory Press, New York, USA, 9. 47-9. 62 and 11.45-11.61]" and the like (for example, hybridization at about 45°C in 6.0xSSC and the like) can be mentioned.

In culturing stem cells such as pluripotent stem cells, it is possible to achieve more stable cultivation of stem cells by using a medium containing a cytokine such as LIF, EGF, or bFGF. Hence, by using a medium containing LIF, EGF, bFGF and the like in the production method of the present invention, it is possible to produce pluripotent stem cells more stably.

LIF can be useful in, for example, maintaining the undifferentiated state of pluripotent stem cells, and EGF and bFGF can be useful in, for example, enhancing the growth of pluripotent stem cells.

The basal medium for the medium used in the production method of the present invention may be any one known per se, without limitation, as long as pluripotent stem cells can be produced by the method of the present invention; for example, DMEM, EMEM, RPMI-1640, α-MEM, F-12, F-10, M-199, HAM, ATCC-CRCM30, DM-160, DM-201, BME, SFM-101, Fischer, McCoy's 5A, Leibovitz's L-15, RITC80-7, HF-C1, MCDB107, NCTC135, Waymouth's MB752/1, StemPro-34 SFM and the like can be mentioned. A medium modified to suit for ES cell culture and the like may be used, and a mixture of the above-described basal medium may be used.

The medium can contain an additive known per se. The additive is not subject to limitation, as long as pluripotent stem cells can be produced by the method of the present invention; for example, growth factors (for example, insulin and the like), iron sources (for example, transferrin and the like), polyamines (for example, putrescine and the like), minerals (for example, sodium selenate and the like), saccharides (for example, glucose and the like), organic acids (for example, pyruvic acid, lactic acid and the like), serum proteins (for example, albumin and the like), amino acids (for example, L-glutamine and the like), reducing agents (for example, 2-mercaptoethanol and the like), vitamins (for example, ascorbic acid, d-biotin and the like), steroids (for example, β-estradiol, progesterone and the like), antibiotics (for example, streptomycin, penicillin, gentamycin and the like), buffering agents (for example, HEPES and the like), nutritive additives (for example, StemPro-Nutrient Supplement and the like) and the like can be mentioned. It is preferable that each of the additives be contained in a concentration range known per se.

Also, the medium can contain a serum. The serum may be any serum derived from an animal, without limitation, as long as pluripotent stem cells can be produced by the method of the present invention, and it is preferably a serum derived from the above-described mammal (for example, fetal calf serum, human serum and the like). A serum substitute additive [for example, Knockout Serum Replacement (KSR) (manufactured by Invitrogen Company) and the like] may be used. The concentration of serum is not subject to limitation, as long as pluripotent stem cells can be produced by the method of the present invention, and it is generally in the range from 0.1 to 30 (v/v) %.

In the production method of the present invention, testis cells may be cultured in the presence of feeder cells. The feeder cells are not subject to limitation, as long as pluripotent stem cells can be produced by the method of the present invention; feeder cells known per se for use in culturing pluripotent stem cells such as ES cells and EG cells while maintaining the pluripotency thereof can be used; for example, fibroblasts (mouse embryonic fibroblasts, mouse fibroblast cell line STO and the like) can be mentioned.

The feeder cells are preferably inactivated by a method known per se, for example, radiation (gamma rays and the like), treatment with an anticancer agent (mitomycin C and the like) and the like.

The cell culture conditions used in the production method of the present invention may be culture conditions in common use in cell culture technology. For example, culture temperature is generally in the range of about 30 to 40°C; preferably about 37°C. The CO₂ concentration is generally in the range of about 1 to 10%, preferably about 5%. Humidity is generally in the range of about 70 to 100%, preferably about 95 to 100%.

Describing in more detail, the method of the present invention of producing pluripotent stem cells is, for example, as follows:

Testis cells separated from the testis are suspended in a medium, sown into a cell culture vessel, and cultured (first culture).

Although the cell culture vessel used may be one for use in ordinary cell culture, it is preferable that the vessel be coated with gelatin and the like to promote the adhesion of testis cells to the vessel. The same applies to the vessels used in the cultures that follow.

Although it is possible to produce pluripotent stem cells solely by continuing the first culture, it is preferable to passage cultured cells in the first culture, preferably non-adherent cultured cells (comprising a reasonable number of germ cells), to another cell culture vessel about 6 to 18 hours after the start of the first culture (for example, after overnight culture) (second culture). The passaged cells form colonies on the base of the cell culture vessel, generally within 1 week after passage, although this time varies depending on culture conditions. The colonization can be confirmed using a microscope and the like.

Preferably, generally 5 to 14 days after the start of the second culture, the cells are dispersed by trypsinization and the like, re-suspended in the medium, and further passaged to a new culture plate (third culture). As passage is repeated in the same way, somatic cells of flat shape disappear. Therefore, after the second or third passage, it is preferable to culture the cells in the presence of feeder cells. The interval of passages and cell dilution rate are determined as appropriate according to culture conditions; for example, an interval of 2 to 5 days and 1 to 1/4 dilution (preferably 1 to 1/2 dilution in the initial stage of culture) can be mentioned. As examples of the interval of passages and cell dilution rate for an established ES-like colony, an interval of 2 to 5 days and 1/4 to 1/10 dilution can be mentioned.

In the above-described culture, the cultured cells form colonies of two different morphologies by about 3 to 6 weeks after the start of culture. One group of colonies have a morphology characterized by an intercellular bridge and morula-like structure, and these are colonies of GS cells. The other group of colonies are more densely packed and have a morphology extremely resembling the morphology of colonies of ES cells, and these are colonies of pluripotent stem cells relating to the present invention. Therefore, the colonies of GS cells and the colonies of pluripotent stem cells relating to the present invention can be clearly distinguished visually.

For example, by selectively picking up a colony of pluripotent stem cells under a microscope using a Pasteur pipette, a micromanipulator and the like, or by limiting dilution and the like, with the above-described morphologies as the indicators, it is possible to isolate pluripotent stem cells. Alternatively, it is possible to isolate the pluripotent stem cells with cell surface markers and the like for the pluripotent stem cells and the like as the indicator, using a cell sorter and the like.

In a mode of embodiment, it is also possible to obtain the pluripotent stem cells of the present invention by culturing testis cells using a medium containing GDNF or an equivalent thereto under the same culture conditions as those described above to obtain GS cells, and continuing to culture the GS cells using a medium containing GDNF or an equivalent thereto under the above-described culturing conditions to , derive pluripotent stem cells from the GS cells.

The morphology of colonies of GS cells, is clearly distinguishable visually from the colonies of the pluripotent stem cells relating to the present invention, as described above; it is possible to isolate GS cells by selectively picking up a colony of GS cells using a Pasteur pipette, micromanipulation and the like under a microscope, or by limiting dilution and the like.

In this case, duration of culture for obtaining GS cells is not subject to limitation, as long as pluripotent stem cells can be produced by the method of the present invention, and the duration of culture is generally within 1 year, for example, within 6 months, preferably within 3 months, more preferably within 7 weeks.

In the method of the present invention of producing pluripotent stem cells, a medium of the same composition may be used throughout the entire process, and a plurality of media of different compositions may be used sequentially. By doing so, it is sometimes possible to grow the pluripotent stem cells more selectively, and to produce the pluripotent stem cells more efficiently.

For example, the medium used for the culture can be changed from the medium used in the initial stage of testis cell culture (designated as medium A) to a medium more suitable for long-term culture of the pluripotent stem cells during the culture.

That is, it is possible to efficiently obtain pluripotent stem cells by culturing testis cells using the medium A to obtain cultured cells, and culturing the cultured cells using the medium B.

The cytokines that can be contained in the medium A are the same as those described above.

Although the medium B may not contain the above-described cytokines [glial cell derived neurotrophic factor (GDNF) or an equivalent thereto, leukemia inhibitory factor (LIF), epidermal growth factor (EGF), basic fibroblast growth factor (bFGF)], it preferably contains leukemia inhibitory factor (LIF) at the same concentrations as those described above.

Also, the concentrations of serum that can be contained in the medium A and medium B, respectively, are the same as those described above; the concentration of serum that can be contained in the medium A is preferably 0.1 to 5 (v/v)%, more preferably 0.3 to 3 (v/v)%. The concentration of serum that can be contained in the medium B is preferably 2 to 30 (v/v)%, more preferably 10 to 20 (v/v)%.

Also, the basal media for the medium A and medium B, respectively are the same as those described above; the basal medium for the medium A can be a basal medium suitably used to culture spermatogonial stem cells (GS cells and the like) (for example, StemPro-34 SFM and the like), and the basal medium for the medium B can be a basal medium suitably used to culture ES cells (for example, DMEM and the like).

The additives the medium A or medium B can contain are the same as those described above.

The timing of changing the medium from the medium A to the medium B is difficult to determine definitely because it varies depending on culture conditions and the like; in the case of mice, for example, the timing is 10 to 120 days, preferably 14 to 40 days, after the start of the first culture.

Furthermore, it is possible to produce pluripotent stem cells at higher efficiency by culturing the cells using a medium of a composition comprising the medium B supplemented with glial cell derived neurotrophic factor (GDNF) or an equivalent thereto at an above-described concentration for about 4 to 40 days just after the medium A was replaced with the medium B.

Such testis cell culture using the medium A and the medium B may be performed in the presence of feeder cells as described above.

The pluripotent stem cells obtained by the production method of the present invention proliferate while maintaining pluripotency generally for 2 months or more, preferably 5 months or more.

In the maintenance, proliferation, and culture of the isolated pluripotent stem cells, the above-described medium B is preferably used.

Whether or not the cells obtained by the production method of the present invention retain pluripotency can be confirmed by a method known per se exemplified below.

For example, the expression of cell surface markers and the like for the cells obtained is analyzed using a flowcytometer and the like. As useful cell surface markers, SSEA-1 (ES cell marker), Forsman antigen (ES cell marker), β1- and α6-integrin (ES and GS cell markers), EpCAM (ES cell and spermatogonia marker), CD9 (ES cell and spermatogonial stem cell marker), EE2 (spermatogonia marker), c-kit (differentiated spermatogonia marker) and the like can be mentioned.

The pluripotent stem cells obtained by the production method of the present invention, for example, mouse-derived pluripotent stem cells, are positive for at least any one selected from the group consisting of SSEA-1, Forsman antigen, β1- and α6-integrin, EpCAM, CD9, EE2 and c-kit, preferably positive for all. Also, they are preferably weakly positive for Forsman antigen and c-kit. Because GS cells are negative for SSEA-1 and Forsman antigen, the pluripotent stem cells obtained by the production method of the present invention are clearly distinguishable from GS cells.

As used herein, "positive" for the expression of a cell surface marker refers to a state wherein the cell surface marker is expressed on the cell surface, and specific binding of a specific antibody for the cell surface marker can be confirmed. "Weakly positive" refers to a state wherein the amount of cell surface marker expressed is relatively weak, a population with less amount of cell surface marker expressed is relatively prevalent, or the ratio of cell population , expressing the cell surface marker is relatively small, compared with other cells, and the like.

In pluripotent stem cells of animal species other than mice, the mode of expression of cell surface markers is the same as with mice. However, provided that a marker exists that is not essentially retained by the animal species, considerations of species differences are made, including the exclusion of the marker from the analysis.

It is also possible to confirm whether or not the cells obtained by the production method of the present invention retain pluripotency by measuring the activity of intracellular alkaline phosphatase in the cells by a method known per se.
The pluripotent stem cells obtained by the production method of the present invention, like ES cells, are positive for alkaline phosphatase. On the other hand, because GS cells are weakly positive to negative for alkaline phosphatase, the pluripotent stem cells obtained by the production method of the present invention are clearly distinguishable from GS cells.

Alternatively, it is also possible to confirm whether or not the cells obtained by the production method of the present invention retain pluripotency by analyzing the expression of a gene specifically expressed in pluripotent stem cells and the like by reverse transcription polymerase chain reaction (RT-PCR) and the like. For example, in the case of mouse-derived pluripotent stem cells, essential molecules for maintaining undifferentiated ES cells, such as Oct-4, Rex-1, Nanog, Cripto, ERas, UTF1, ZFP57, and Esg-1, can be mentioned as examples of the gene specifically expressed in pluripotent stem cells. The pluripotent stem cells obtained by the production method of the present invention express at least any gene selected from the group consisting of Oct-4, Rex-1, Nanog, Cripto, ERas, UTF1, ZFP57 and Esg-1, and preferably express all these genes. In GS cells, the expression of these genes is generally weaker than that in the pluripotent stem cells obtained by the production method of the present invention, and particularly almost no expression of Nanog is observed; the pluripotent stem cells are clearly distinguishable from GS cells.

Furthermore, it is also possible to confirm whether or not the cells obtained by the production method of the present invention retain pluripotency, or to clearly distinguish the cells from other stem cells (ES cells, GS cells and the like) by analyzing the imprinting pattern of the cells by bisulfite genomic sequencing of DMRs in chromosome DNA (Development, vol.129, p1807-1817, 2002), COBRA (Nucl. Acid. Res., vol.25, p2532-2534, 1997) and the like. For example, provided that the pluripotent stem cells obtained by the method of the present invention are of mouse origin, the DMRs of Igf2r and Peg10, which are the maternal imprint regions, are hardly methylated, whereas those of ES cells are more methylated (for example, 2 times or more as the frequency of methylation). Also, the DMRs of the H19 and Meg3IG, which are paternal imprint regions, is nearly completely methylated in GS cells, whereas in the pluripotent stem cells of the present invention, the methylation thereof is incomplete (for example, 0 to 60% as the frequency of methylation).

Because the DMRs of GS cells can have a nearly completely male imprinting pattern, it is possible to monitoring the ratio of pluripotent stem cells in the culture and the degree of progression of the production by tracking the imprinting pattern in producing the pluripotent stem cells from spermatogonial stem cells (GS cells and the like) using the method of the present invention. That is, with the derivation of pluripotent stem cells from GS cells, the frequency of methylation of DMRs in paternal imprinting regions (H19, Meg3IG, Rasgfr1 and the like) can decrease.

It is also possible to confirm that the pluripotent stem cells produced by the method of the present invention maintain the undifferentiated state by confirming a low methylation state (for example, a state wherein the frequency of methylation is not more than 20%) of the DMR in the Oct-4 , region.

It is also possible to confirm the pluripotency of the cells obtained by the production method of the present invention by injecting the cells into the subcutaneous tissue, seminiferous tubule and the like of an immunodeficient animal or an animal with immune tolerance induced therein, and analyzing for the presence or absence of the formation of teratomas. The pluripotent stem cells obtained by the production method of the present invention are capable of forming teratomas; diverse cells differentiated into the three germ layers (for example, nerves, epidermis, muscles, bronchial epithelium, cartilages, bones, squamous epithelium, neuroepithelium and the like) are found in the teratomas. On the other hand, GS cells form spermatogenic colonies but do not form teratomas when injected into the seminiferous tubule. Therefore, the pluripotent stem cells obtained by the production method of the present invention are clearly distinguishable from GS cells.

It is also possible to confirm whether or not the cells obtained by the production method of the present invention retain pluripotency by introducing the cells into host embryos, and analyzing for the presence or absence of the birth of a chimeric animal. The pluripotent stem cells obtained by the production method of the present invention are capable of contributing to the normal development of a chimeric animal when introduced into host embryos. On the other hand, GS cells are incapable of contributing to the normal development of a chimeric animal even when introduced into host embryos; therefore, the pluripotent stem cells obtained by the production method of the present invention are clearly distinguishable from GS cells.

It is also possible to confirm the pluripotency of the cells obtained by the production method of the present invention by applying a method known per se for differentiating ES or EG cells into various functional cells in vitro, and analyzing the differentiation capacity in vitro of the cells. "Functional cells" are somatic cells or germ cells that can be derived from ES or EG cells; for example, ectodermal cells, mesodermal cells, endodermal cells and the like can be mentioned.

For example, the pluripotent stem cells obtained by the production method of the present invention differentiate into mesodermal cells when cultured under mesodermal cell differentiation conditions known per se.

Examples of mesodermal cells include, but are not limited to, blood cell lineage cells (including hematopoietic lineage cells), vascular lineage cells (vascular endothelial cells and the like), myocardial cells (for example, atrial muscle cells, ventricular muscle cells and the like), osteocytes, chondrocytes, tendon cells, adipocytes, skeletal muscle cells, smooth muscle cells and the like. Preferably, the mesodermal cells are blood cell lineage cells, vascular lineage cells (vascular endothelial cells and the like) or myocardial cells.

Examples of the above-described blood cell lineage cells include, but are not limited to, blood cells (for example, CD45-positive cells and the like), erythroblast lineage cells (for example, Ter119-positive cells and the like), myeloid lineage cells [for example, monocyte lineage cells (for example, MAC1-positive cells and the like), neutrophil lineage cells (for example, Gr1-positive cells and the like)] and the like.

As examples of the above-described myocardial cells, MF20-positive cells and the like, cTn-I-positive cells and the like can be mentioned; as atrial muscle cells, ANP-positive cells and the like can be mentioned; as ventricular muscle cells, MLC2v-positive cells and the like can be mentioned; as examples of the above-described vascular lineage cells (vascular endothelial cells and the like), CD31-positive cells, VE cadherin-positive cells and the like can be mentioned. Vascular lineage cells can also be identified by uptake of DiI-acetylated low-density lipoprotein.

The mesodermal cell differentiation conditions include, but are not limited to, conditions known per se that allow ES or EG cells to differentiate into mesodermal cells; for example, culture in a plate coated with type IV collagen (see, for example, Blood, vol.93, p1253-1263, 1999 and the like), culture in methylcellulose medium (Development, vol 125, p1747-1757, 1998), co-culture with feeder cells for inducing mesodermal cell differentiation (for example, stroma cells such as OP9 cells) (see Proc. Natl. Acad. Sci. USA, vol.100, p4018-4023, 2003; Exp. Hematol., vol.22, p979-984; Science, vol.272, 722-724, 1996; Blood, vol.93, p1253-1263, 1999; Development, vol 125, p1747-1757, 1998 and the like) and the like can be mentioned.

When the pluripotent stem cells obtained by the production method of the present invention are differentiated into blood cell lineage cells or vascular lineage cells (vascular endothelial cells and the like), it is desirable that the pluripotent stem cells be co-cultured with the above-described feeder cells for inducing mesodermal cell differentiation (see, for example, "Proc. Natl. Acad. Sci. USA, vol.100, p4018-4023, 2003", "Exp. Hematol., vol.22, p979-984", "Science, vol.272, 722-724, 1996" and the like). In the case of mice, for example, it is possible to obtain vascular cells by co-culturing the pluripotent stem cells with the above-described feeder cells for inducing mesodermal cell differentiation to induce differentiation into vasculo-hematopoietic precursor cells, collecting the cells as, for example, PECAM-1-positive cells or Flk-1-positive cells, and further co-culturing the obtained cells with feeder cells for inducing mesodermal cell differentiation. Alternatively, the cells may be cultured in methylcellulose medium (Development, vol 125, p1747-1757, 1998).

When the pluripotent stem cells obtained by the production method of the present invention are differentiated into myocardial cells, the pluripotent stem cells are suitably co-cultured with the above-described feeder cells for inducing mesodermal cell differentiation in the presence of SCF (see, for example, Proc. Natl. Acad. Sci. USA, vol.100, p4018-4023, 2003 and the like). Alternatively, in the case of mice, for example, it is possible to obtain myocardial cells by co-culturing the pluripotent stem cells with the above-described feeder cells for inducing mesodermal cell differentiation, collecting Flk-1-positive cells, and further co-culturing the obtained cells with feeder cells for inducing mesodermal cell differentiation.

Also, the pluripotent stem cells obtained by the production method of the present invention differentiate into ectodermal cells when cultured under ectodermal cell differentiation conditions known per se. Examples of the ectodermal cells include, but are not limited to, neuronal lineage cells, epidermal lineage cells and the like. The ectodermal cell differentiation conditions include, but are not limited to, conditions known per se that allow ES or EG cells to differentiate into ectodermal cells; for example, the following neuronal lineage cell differentiation induction conditions and the like can be mentioned.

The pluripotent stem cells obtained by the production method of the present invention differentiate into neuronal lineage cells when cultured under neuronal lineage cell differentiation conditions known per se. As examples of the neuronal lineage cells, neurons (for example, MAP2-positive cells, Tuj-positive cells and the like), dopaminergic neurons (for example, cells positive for both TH and Tuj and the like), glial cells (for example, MBP-positive cells and the like), oligodendrocytes (for example, MBP-positive cells and the like), astrocytes (for example, GFAP-positive cells and the like) and the like can be mentioned.

The neuronal lineage cell differentiation conditions include, but are not limited to, conditions known per se that allow ES or EG cells to differentiate into neuronal lineage cells; for example, culture on a gelatin-coated plate using a medium for inducing neuronal differentiation (for example, N2B27 medium) and the like can be mentioned (see, for example, Nature Biotechnology, vol.21, 183-186, 2003 and the like).

Also, the pluripotent stem cells obtained by the production method of the present invention differentiate into endodermal cells when cultured under endodermal cell differentiation conditions known per se. The endodermal cells include, but are not limited to, gastrointestinal lineage cells, pancreas cells, hapatocytes, respiratory lineage cells, thyroid and the like. The endodermal cell differentiation conditions include, but are not limited to, conditions known per se that allow ES or EG cells to differentiate into endodermal cells; for example, conditions for differentiation into insulin-producing cells (Proc Natl Acad Sci USA, 97, 11307-11312) and the like can be mentioned.

The pluripotent stem cells obtained by the production method of the present invention can be cryopreserved semi-permanently, and can be used after thawing and awakening from dormancy as required. The pluripotent stem cells maintain pluripotency even after cryopreservation and thawing. In the cryopreservation, the cells are suspended in a composition for cryopreservation of cells known per se, such as Cell Banker (manufactured by DIA-IATRON Company), which comprises dimethylsulfoxide and fetal calf serum albumin, and the cells are preserved under conditions of -80 to -200°C, preferably -196°C (in liquid nitrogen).

If the pluripotent stem cells obtained by the production method of the present invention are awaken from dormancy after cryopreservation, the cells are thawed in a solvent according to a conventional method and suspended to yield a cell suspension. The method of thawing is not subject to limitation; for example, thawing can be performed in a 37°C thermostat bath using a DMEM containing 10% fetal calf serum, (DMEM/FCS). Specifically, the freezing tube is floated in the thermostat bath, and DMEM/FCS is added drop by drop to the frozen cells to thaw the cells. After the cells are centrifuged and washed, they are re-suspended in the medium.

Even when the pluripotent stem cells once awakened from dormancy are cultured and then again frozen, the pluripotency of the cells is maintained.

Because the pluripotent stem cells obtained by the production method of the present invention are capable of proliferating over a long period while maintaining their pluripotency, it is possible to modify a gene of the pluripotent stem cells by a method known per se, and to produce genetically modified pluripotent stem cells, for example, pluripotent stem cells transfected with a particular exogenous gene, pluripotent stem cells lacking a particular gene, and the like.

As examples of the method of introducing an gene to pluripotent stem cells produced by the method of the present invention, a method comprising introducing a vector constructed to allow the functional expression of a particular gene to spermatogonial stem cells can be mentioned. As the vector, a plasmid vector, a viral vector and the like can be used. Additionally, as the viral vector, retrovirus, adenovirus, lentivirus, herpesvirus, adeno-associated virus, parvovirus, Semliki forest fever virus, vaccinia virus and the like can be mentioned.

As examples of the method of introducing a vector to pluripotent stem cells, common gene transfection methods such as the calcium phosphate method, the DEAE dextran method, the electroporation method, or the lipofection method can be mentioned. When using a virus as the vector, the virus' genome may be introduced to cells by one of the above-described common gene transfection methods, and the virus' genome can also be introduced to cells by infecting the cells with virus particles. ,

For selecting genetically modified pluripotent stem cells stably incorporating an particular extraneous gene, a marker gene, simultaneously with a vector, may be introduced to these cells, and the cells may be cultured by a method suitable for the properties of the marker gene. For example, when the marker gene is a gene that confers drug resistance to a selection drug that is lethal to the host cells, the spermatogonial stem cells incorporating a vector may be cultured using a medium supplemented with the drug. As examples of the combination of a drug-resistance-conferring gene and a selection drug, a combination of the neomycin-resistance-conferring gene and neomycin (G418), a combination of the hygromycin-resistance-conferring gene and hygromycin, a combination of the blasticidin-S-resistance-conferring gene and blasticidin S, and the like can be mentioned.

As an example of the method of obtaining pluripotent stem cells lacking a particular gene, homologous recombination using a targeting vector (gene targeting method) can be mentioned. Specifically, pluripotent stem cells lacking a particular gene can be obtained by isolating the chromosome DNA of the particular gene; introducing, to the chromosome of pluripotent stem cells by the homologous recombination method, a DNA strand (targeting vector) having a DNA sequence constructed to destroy the gene by inserting, to an exon portion of the gene, a drug resistance gene represented by the neomycin resistance gene or the hygromycin resistance gene, a reporter gene represented by *lacZ* (β-galactosidase gene), *cat* (chloramphenicol acetyltransferase gene) and the like to destroy the exon function, by inserting a DNA sequence that terminates gene transcription to the intron portion between exons (for example, polyA addition signal and the like) to prevent the synthesis of complete messenger RNA, and the like; analyzing the thus obtained cells by Southern hybridization analysis using a DNA sequence in the DNA of a particular gene or a DNA sequence in the vicinity of the DNA as a probe or by a PCR method with primers of the DNA sequence in the targeting vector and a DNA sequence in the vicinity of, but other than, the DNA of the particular gene used to prepare the targeting vector; and selecting pluripotent stem cells lacking the particular gene. Alternatively, the Cre-loxP system, which deletes a particular gene in a tissue-specific or developmental-stage-specific manner, and the like may also be used (Marth, J.D. (1996) Clin. Invest. 97:1999-2002; Wagner, K.U. et al. (1997), Nucleic Acids Res. 25:4323-4330).

The pluripotent stem cells obtained by the production method of the present invention have the capability of differentiating into all somatic cells constituting a living organism; all experimental techniques and methods applicable to ES cells or EG cells can be applied to the pluripotent stem cells; using the pluripotent stem cells, it is possible to produce diverse functional cells, tissues, animals (excluding humans) and the like. Provided that pluripotent stem cells genetically modified by the above-described method are used, it is possible to produce genetically modified diverse functional cells, tissues, animals (excluding humans) and the like.

For example, it is possible to produce the above-described mesodermal cells by culturing the pluripotent stem cells obtained by the production method of the present invention under the above-described mesodermal cell differentiation conditions.

Also, it is possible to produce the above-described ectodermal cells (for example, neuronal lineage cells) by culturing the mouse pluripotent stem cells obtained by the production method of the present invention under the above-described endodermal cell (for example, neuronal lineage cells and the like) differentiation conditions.

Furthermore, it is possible to produce the above-described endodermal cells by culturing the pluripotent stem cells obtained by the production method of the present invention under the above-described endodermal cell differentiation conditions.

Additionally, it is possible to produce diverse functional cells by inducing the pluripotent stem cells obtained by the production method of the present invention to differentiate into diverse functional cells using, for example, a method of differentiating ES cells into vascular cells (vascular endothelial cells and the like) (Development, vol.125, 1747-1757, 1998), a method of differentiating ES cells into nerve cells (Neuron, vol.28, 31-40, 2000), a method of differentiating ES cells into pigment cells (Development, vol.125, 2915-2923, 1998), a method of differentiating ES cells into insulin-producing cells (Proc Natl Acad Sci USA, 97, 11307-11312, 2000), a method of differentiating ES cells into ectodermal cells (pamphlet for WO01/088100), a method of producing endodermal cells, ectodermal cells, mesodermal cells, blood cells, endothelial cells, chondrocytes, skeletal muscle cells, smooth muscle cells, myocardial cells, glial cells, neurons, epithelial cells, melanocytes, or keratinocytes by forming an embryoid body of ES cells (Reprod. Fertil. Dev., 10, 31, 1998) and the like. Alternatively, it is also possible to produce germ cells such as spermatozoa from the pluripotent stem cells of the present invention by the methods described in Proc Natl Acad Sci USA, vol.100, p11457-11462, 2003 and Nature, vol.427, p148-154, 2004, and to obtain offspring animals of the pluripotent stem cells by using the germ cells for crossing.

It is also possible to transfer the pluripotent stem cells obtained by the production method of the present invention to an immunodeficient animal such as a nude mouse, or to an animal with immune tolerance induced therein, to form teratomas, and to isolate diverse functional cells from the teratomas.

Furthermore, it is possible to obtain genetically modified functional cells by modifying a gene in the pluripotent stem cells obtained by the production method of the present invention, and applying the above-described method to the genetically modified pluripotent stem cells obtained.

Production of an animal (excluding humans) using the pluripotent stem cells relating to the present invention can be performed in accordance with, for example, a method known per se such as a method using a chimeric embryo.

For example, first, a pluripotent stem cell obtained by the production method of the present invention is introduced into a host embryo to obtain a chimeric embryo. The animal species of the "host" is preferably the same as the animal species of the pluripotent stem cell introduced. Examples of the "embryo" include, but are not limited to, blastocysts, 8-cell stage embryos and the like.

An "embryo" can be obtained by mating a female animal that received a superovulation treatment with a hormone preparation (for example, PMSG, which has FSH-like action, and hCG, which has LH action, are used) and the like with a male animal and the like. As methods of introducing a pluripotent stem cell into a host embryo, the microinjection method, aggregation method and the like are known, and any method can be used.

Next, the chimeric embryo is transferred to the uterus or oviduct of the host animal to obtain a chimeric animal (excluding humans). The host animal is preferably a pseudo-pregnant animal. A pseudo-pregnant animal can be obtained by mating a female animal in the normal sexual cycle with a male animal emasculated by vasoligation and the like. The host animal having the transferred chimeric embryo will become pregnant and bear a chimeric animal (excluding humans).

Furthermore, it is possible to obtain an animal (excluding humans) harboring the gene derived from the pluripotent stem cells (an animal derived from the pluripotent stem cells) by mating the chimeric animal (excluding humans) with a normal animal or within the chimeric animals, and selecting an individual harboring the gene derived from the pluripotent stem cells from among the individuals of next , generation (F1). In selecting an animal (excluding humans) harboring a gene derived from pluripotent stem cells, various characters can be used as indicators; for example, body color and coat color are used as the indicators. It is also possible to perform the selection by extracting DNA from a portion of the body and performing Southern blot analysis or PCR assay.

It is also possible to directly obtain an animal derived from the pluripotent stem cells relating to the present invention by introducing the pluripotent stem cells into a tetraploid embryo to obtain a tetraploid chimeric embryo, and transferring the tetraploid chimeric embryo into the uterus or oviduct of a host animal, (Proc. Natl. Acad. Sci. USA, vol.90, p8424-8428, 1993). Although a tetraploid embryo can be obtained by electrofusing blastocysts by a method known per se, it is also possible to achieve electrofusion by applying electric pulses to 2-cell blastocysts in mannitol solution.

By using the above-described method, for example, it is possible to obtain an animal (transgenic animal) harboring a particular exogenous gene from pluripotent stem cells transfected with the exogenous gene. Also, from pluripotent stem cells lacking a particular gene, it is possible to obtain a gene-deficient heterozygotous animal. Furthermore, by propagating the gene-deficient heterozygotous animals obtained, it is possible to obtain a gene-deficient homozygotous animal.

The present invention also relates to a composition for producing pluripotent stem cells derived from testis cells, which contains glial cell derived neurotrophic factor (GDNF) or an equivalent thereto. By culturing testis cells by the above-described method using a medium containing the composition, it is possible to obtain pluripotent stem cells derived from the testis cells.

The composition can further contain leukemia inhibitory factor (LIF).

Also, the composition can further contain at least one of epidermal growth factor (EGF) and basic fibroblast growth factor (bFGF), preferably all.

The composition can further contain a physiologically acceptable carrier, excipient, antiseptic, stabilizer, binder, solubilizer, nonionic surfactant, buffering agent, preservative, antioxidant, the above-described additive, basal medium and the like.

The composition is used in the form of an isotonic aqueous solution or powder and the like, added to the medium used in the production method of the present invention and otherwise. Alternatively, the composition may be a medium used for the production method of the present invention.

The present invention is explained in more detail in the following by referring to Examples, which are not to be construed as limitative.

### Examples

### Materials and Methods

### (Cell Culture)

Testis cells were collected from newborn (0-8 days old) ddY mice, DBA/2 mice or transgenic mouse line C57BL6/Tg14(act-EGFP-Osby01) that was bred into DBA/2 background (designated Green) (provided by Dr. M. Okabe, Osaka University). Because these Green mice have the expressed the EGFP gene in substantially all cell types, it is possible to track the cells derived from the mice can be tracked with the fluorescence of EGFP as the indicator.

For some experiments, testis cells were collected from a newborn p53 deficient mouse in ICR background (Oncogene, vol.8, p3313-3322, 1993).

Testis cells were collected by two-step enzymatic digestion using collagenase (type IV, Sigma) and trypsin (Invitrogen).

That is, the mouse testis was extirpated, the tunica albuginea was removed in PBS, and incubation was performed in Hunks' balanced solution containing 1 mg/ml collagenase (type I) at 37°C for 15 minutes with shaking as appropriate to loosen the seminiferous tubule. After removal of the non-adherent interstitial cells by two times of washing with PBS, incubation was performed in a 0.25% trypsin solution containing 1.4 mg/ml DNase at 37°C for 15 minutes with shaking as appropriate to disassemble the seminiferous tubule. After PBS was added to inactivate the trypsin, pipetting was performed to obtain a cell suspension. This was passed through 20- to 30-µm nylon meshes to remove the undigested cell mass, centrifugation was performed at 600 x g for 5 minutes, and testis cells were recovered.

Testis cells were allocated to a gelatin-coated tissue culture plate (2 × 10⁵ cells/3.8 cm²). Culture medium for the testis cells was StemPro-34 SFM (Invitrogen) supplemented with StemPro supplement (Invitrogen), 25 µg/ml Insulin, 100 µg/ml transferrin, 60 µM putrescine, 30 nM sodium selenite, 6 mg/ml D-(+)-glucose, 30 µg/ml pyruvic acid, 1 µl/ml DL-lactic acid (Sigma), 5 mg/ml bovine albumin (ICN Biomedicals), 2 mM L-glutamine, 5×10⁻⁵ M 2-mercaptoethanol, MEM non-essential vitamin solution (Invitrogen), 10⁻⁴M ascorbic acid, 10 µg/ml d-biotin, 30 ng/ml β-estradiol, 60 ng/ml progesterone (Sigma), 20 ng/ml mouse epidermal growth factor (EGF: Becton Dickinson), 10 ng/ml basic fibroblast growth factor (bFGF:Becton Dickinson), 10³ units/ml ESGRO (mouse leukemia inhibitory factor: LIF, Invitrogen), 10 ng/ml recombinant rat GDNF (R&D Systems), and 1 (v/v)% fetal calf serum (JRH Biosciences). The cells were maintained at 37°C in an atmosphere of 5% carbon dioxide in air.

After overnight incubation, the floating cells were passaged to secondary culture plates after vigorous pipetting. Within 1 wk, the passaged cells proliferated, spread on the bottom of the plate, and formed colonies.

Cells were dispersed by trypsin treatment and transferred at intervals of 5-14 days (this interval is called "DIV" for short) to a fresh culture plate (x 1-1/2 dilution) *in vitro*. Colonies grew to the original size in about 10 days, and cells were again passaged (× 1 dilution). From the second or third passage, the cells were maintained on mitomycin C-inactivated mouse embryonic fibroblasts (MEF) and were passaged to new MEF, at 1 to 1/2 fold dilution in the initial stage of cultivation, and at 1 to 1/4 fold dilution thereafter, every 2 to 5 days. Furthermore, the established ES cell-like colony was passaged to new MEF at 1/4 to 1/10 fold dilution, every 2 to 5 days.

After appearance of ES cell like colonies, the cells were cultured in Dulbecco's modified Eagle's medium supplemented with 15 (v/v)% FCS, 0.1 mM 2-mercaptoethanol, 10³ units/ml ESGRO (mouse leukemia inhibitory factor, Invitrogen) and 10 ng/ml recombinant rat GDNF (R&D Systems), at a final concentration.

Subsequently, the cells were maintained in Dulbecco's modified Eagle's medium supplemented with 15 (v/v)% FCS, 0.1 mM 2-mercaptoethanol, and 10³ units/ml ESGRO (mouse leukemia inhibitory factor, Invitrogen), at a final concentration.

In some experiments, after appearance of ES cell like colonies, the cells were cultured and maintained in Dulbecco's modified Eagle's medium supplemented with 15 (v/v)% FCS, 0.05 mM 2-mercaptoethanol and 10³ units/ml ESGRO (mouse leukemia inhibitory factor, Invitrogen), at a final concentration (said culture conditions are sometimes to be referred to as ES cell culture conditions).

To induce EG cells from neonatal testis, the same medium was also supplemented with 20 ng/ml human bFGF (Invitrogen), and cells were cultured on Sl⁴-m220 (gift from Dr. T. Nakano, Osaka University).

For adult testis culture, 2 × 10⁷ cells from 3- to 8-week-old p53 knockout mice were used to recover spermatogonial stem cells with anti-CD9 antibody as described elsewhere (Biol. Reprod., vol.70, p70-75, 2004), and selected cells were plated on gelatin-coated plate (3 × 10⁵ cells/9.5 cm²). GS cell colonies were picked by micromanipulation and transferred to, MEF for expansion.

ES-like cells and GS cells could also be separated by picking up colonies with a Pasteur pipette and the like under a stereoscopic microscope.

For differentiation into mesodermal lineages, the cultured cells were cultured on OP9 feeder layers, and cell differentiation was carried out as described (Science, vol.272, p722-724, 1996, Development, vol.125, p1747-1757, 1998; Proc. Natl. Acad. Sci. USA, vol.100, p4018-4023, 2003; Blood, vol.93, p1253-1263, 1999 and the like). All cytokines used for the differentiation were provided by Kirin Brewery.

Induction of differentiation of cultured cells into blood cell lineage cells in vitro was performed as described in Science, vol.272, p722-724, 1996. That is, by culturing the cultured cells on the OP9 stroma feeder, differentiation into blood cell lineage cells was induced.

Also, in some experiments, induction of differentiation of cultured cells into blood cell lineage cells was performed as described in Development, vol 125, p1747-1757, 1998. That is, the cultured cells were cultured in methylcellulose medium.

Induction of differentiation of cultured cells into myocardial cells in vitro was performed as described in Proc. Natl. Acad. Sci. USA, vol.100, p4018-4023, 2003. That is, by culturing the cultured cells in the presence of SCF on the OP9 stroma feeder, differentiation into myocardial cells was induced.

Induction of differentiation of cultured cells into vascular cells (vascular endothelial cells and the like) in vitro was performed as described in Proc. Natl. Acad. Sci. USA, vol.100, p4018-4023, 2003. That is, by culturing the cultured cells on the OP9 stroma feeder, differentiation into vasculo-hematopoietic precursor cells was induced, sorting PECAM-1-positive cells 5 days later, and further culturing the sorted cells on the OP9 stroma feeder, differentiation into vascular cells (vascular endothelial cells and the like) was induced. ,

Vascular cells were identified by the uptake of DiI-acetylated low-density lipoprotein (Molecular Probes).

Induction of differentiation of cultured cells into neurons and glial cells in vitro was performed using N2B27 medium as described in Nature Biotechnology, vol.21, p183-186, 2003. In brief, the cultured cells were plated onto 0.1% gelatin-coated tissue culture plastic at a density of 0.5-1.5 x 10⁴ / cm² in N2B27 medium. Medium renewed every 2 days. N2B27 is a 1:1 mixture of DMEM/F12 (Sigma) supplemented with modified N2 (25 g/ml insulin, 100 g/ml apo-transferrin, 6 ng/ml progesterone, 16 g/ml putrescine, 30 nM sodium selenite and 50 g/ml bovine serum albumin fraction V (Gibco)) and Neurobasal medium supplemented with B27 (both from Gibco).

ES cells derived from 129svj mice were used. In some experiments, D3 ES cells that ubiquitously express the EGFP gene under the CAG promoter were used (provided by Dr. M. Okabe, Osaka University; Gene, vol.108, p193-200, 1991). ES cells were maintained in standard ES cell medium.

### (Antibodies and staining)

To confirm the properties of cells produced by the production method of the present invention and the like, flowcytometry was performed to examine for the expression of markers for ES cells, spermatogenic cells and the like known per se.

As primary antibodies, rat anti-EpCAM (G8.8), mouse anti-SSEA-1 (MC-480), mouse anti-sarcomeric protein (MF20; Developmental Studies Hybridoma Bank, University of Iowa), rat anti-mouse Forssman antigen (M1/87), rat anti-human a6-integrin (CD49f) (GoH3), biotinylated hamster anti-rat β1-integrin (CD29) (Ha2/5), biotinylated rat anti-mouse CD9 (KMC8), APC-conjugated rat anti-mouse c-kit (2B8), rat anti-mouse CD31 (MEC 13.3), PE-conjugated rat anti-mouse Ter119 (Ter-119), biotinylated rat anti-mouse Mac1 (M1/70), biotinylated rat anti-mouse Gr1 (RB6-8C5), rat anti-mouse VE-cadherin (11D4.1), APC-conjugated rat anti-mouse CD45 (30-F11; BD Biosciences), rat anti-TDA (EE2; provided by Dr. Y. Nishimune, Osaka University), APC-conjugated rat anti-mouse Flk-1 (Avas 12a1; provided by Dr. S. Nishikawa, RIKEN), goat anti-mouse cardiac troponin-I (cTn-I) (Santa Cruz Biotechnology), mouse anti-human myosin light chain 2v (MLC2v) (Alexis Biochemicals Inc), rabbit anti-mouse atrial natriuretic peptide (ANP) (Protos Biotech Corporation), mouse anti-human myelin basic protein (MBP) (Pm43), rabbit anti-glial fibrillary acidic protein (GFAP), rabbit anti-mouse tyrosine hydroxylase (TH), mouse anti-human β-tubulin III (Tuj) (SDL.3D10) (Sigma), anti-MAP2 rabbit polyclonal antibody, and mouse anti-myosin heavy chain monoclonal antibody (MF20) were used.

APC-conjugated goat anti-rat-IgG (Cedarlane Laboratories), APC-conjugated streptavidin (BD Biosciences), Alexa Fluor 488-conjugated goat anti-mouse IgG, Alexa Fluor 647-conjugated goat anti-rat IgM, Alexa Fluor 633-conjugated goat anti-mouse IgM (Molecular Probes), Cy3-conjugated donkey anti-mouse IgG, Cy3-conjugated donkey anti-rabbit IgG, ALP or peroxidase-conjugated donkey anti-mouse IgG, ALP-conjugated donkey anti-rabbit IgG (Jackson Immunoresearch), ALP-conjugated rabbit anti-goat IgG (Vector Laboratories, Burlingame), or ALP-conjugated goat anti-rat IgG (Chemicon) were used as secondary antibodies.

The cell staining was carried out according to Proc Natl Acad Sci USA, vol.96, p5504-5509, 1999. Cells were analyzed with a FACSCalibur system (BD Biosciences).

Immunocytochemistory for functional cells differentiated in vitro was carried out using standard protocols. Cells were fixed in 4% paraformaldehyde in PBS and treated with primary antibodies. Localization of antigens was visualized by using secondary antibodies conjugated with Cy3.

ALP or DAB staining was carried out using a VECTOR alkaline phosphatase substrate kit or DAB substrate kit (Vector Laboratories), respectively, according to manufacturer's , protocol.

Alkaline phosphatase staining was carried out according to Nature, vol.352, 809-811, 1991, and Cell, vol.44, 831-838, 1986.

### (Transplantation and analysis of recipients)

In the analysis of teratoma formation, approximately 2 × 10⁶ cultured cells were injected subcutaneously into KSN nude mice (Japan SLC), and analyzed 3 weeks after transplantation. Formed tissues were fixed in 10% neutral-buffered formalin and processed for paraffin sectioning. Sections were stained with hematoxylin and eosin, and examined under the microscope.

For microinjections into the seminiferous tubules, approximately 3 × 10⁵ cells were injected into the seminiferous tubules of an immune-suppressed W mouse (Japan SLC) recipient through the efferent duct (Biol. Reprod., vol.68, 167-173, 2003) .

All animal experimentation protocols were approved by the Institutional Animal Care and Use Committee of Kyoto University.

### (Chimera Formation and Microinsemination)

10-15 cultured cells derived from Green mice were injected into the blastocoel of 3.5 dpc blastocysts of C57BL/6 mice using a Piezo-driven micromanipulator(Development, vol.121, 2397-2405, 1995). The blastocysts were returned to the oviducts or uteri of 2.5 dpc pseudopregnant ICR foster mothers on the day of microinjection. Approximately 70% of the cells retained the euploid karyotype at the time of injection, which significantly influences the chimerism rate or germline transmission after ES cell injection.

Tetraploid embryo aggregation chimeras were produced using the method developed by Nagy et al. (Proc. Natl. Acad. Sci. USA, vol.90, p8424-8428, 1993)), except that two-cell blastomeres were electrofused by applying an electric pulse (2500 V/cm, 10 µsec) in 300 mM mannitol solution.

Fetal mice at 12.5 dpc were extirpated and examined using a stereoscopic microscope under UV light. Also, the fetal mice were fixed in 4% para-formaldehyde and frozen in the Tissue-Tek OCT compound (Sakura Fine Technical Co., Ltd.), and frozen sections were prepared. The sections were analyzed for chimerism with the fluorescence of EGFP derived from Green mice using a fluorescent microscope (OLYMPUS confocal laser scanning microscope). PI was used as the control stain.

Newborn chimeric mice born by spontaneous delivery were examined using a stereoscopic microscope under UV light.

Microinsemination was performed using BDF1 oocytes as described (Development, vol.121, p2397-2405, 1995). Embryos were transferred on the day after the cultivation.

### (RT-PCR)

Expression analysis for Oct-4, HPRT, Rex-1, Nanog, ERas, Esg-1, Cripto and ZFP57by RT-PCR were carried out using specific primers, as described (Science, vol.297, 392-395, 2002; Mol. Cell. Biol., vol.13, 473-486, 1993; Cell, vol.113, 631-642, 2003; PNAS, vol.100, 14926-14931, 2003; Nature, vol.423, 541-545, 2003; Genome Res., vol.12, 1921-1928, 2002; Dev. Biol., vol.235, 12-32, 2001; Dev. Biol., vol.265, 491-501, 2004 and the like). PCR amplifications for Oct-4, UTF1, and HPRT were carried out by using following specific primers.
[Oct-4]
5'-AGCTGCTGAAGCAGAAGAGG-3' (SEQ ID NO: 1)
5'-GGTTCTCATTGTTGTCGGCT-3' (SEQ ID NO: 2)
[UTF1]
5'-GATGTCCCGGTGACTACGTCT-3' (SEQ ID NO: 3)
5'-TCGGGGAGGATTCGAAGGTAT-3' (SEQ ID NO: 4)
[HPRT]
5'-GCTGGTGAAAAGGACCTCT-3' (SEQ ID NO: 5)
5'-CACAGGACTAGAACACCTGC-3' (SEQ ID NO: 6)

### (Analysis of Imprinted Genes)

Bisulfite genomic sequencing of DMRs of imprinted genes was carried out as described (Development, vol.129, p1807-1817, 2002). PCR amplifications of each DMR region from bisulfite-, treated genomic DNAs were carried out by using following specific primers.
[H19]
5'-GGAATATTTGTGTTTTTGGAGGG-3' (SEQ ID NO: 7)
5'-AATTTGGGTTGGAGATGAAAATATTG-3' (SEQ ID NO: 8)
[Meg3 IG]
5'-GGTTTGGTATATATGGATGTATTGTAATATAGG-3' (SEQ ID NO: 9)
5'-ATAAAACACCAAATCTATACCAAAATATACC-3' (SEQ ID NO: 10)
[Rasgrfl]
5'-GTGTAGAATATGGGGTTGTTTTATATTG-3' (SEQ ID NO: 11)
5'-ATAATACAACAACAACAATAACAATC-3' (SEQ ID NO: 12)
[Igf2r]
5'-TTAGTGGGGTATTTTTATTTGTATGG-3' (SEQ ID NO: 13)
5'-AAATATCCTAAAAATACAAACTACACAA-3' (SEQ ID NO: 14)
[Peg10]
5'-GTAAAGTGATTGGTTTTGTATTTTTAAGTG-3' (SEQ ID NO: 15)
5'-TTAATTACTCTCCTACAACTTTCCAAATT-3' (SEQ ID NO: 16)
[Oct-4]
5'-GGTTTTTTAGAGGATGGTTGAGTG-3' (SEQ ID NO: 17)
5'-TCCAACCCTACTAACCCATCACC-3' (SEQ ID NO: 18)

The DNA sequences were determined in both directions. For COBRA, PCR products were digested with restriction enzymes with a recognition sequence containing CpG in the original unconverted DNA (Nucl. Acid. Res., vol.25, 2532-2534, 1997). Intensity of digested DNA bands was quantified with ImageGauge software (Fuji Photo Film).

### Results

When neonatal DBA/2 or ddY mouse testis cells were cultured in a medium containing GDNF, bFGF, EGF, and LIF, the majority of the colonies that had the typical appearance of GS cells, which are characterized by intercellular bridges and a morula-like structure (Figure 1d). However, a few colonies (<5%) were remarkably similar to ES cells (Figure 1a and 1b). These colonies were more tightly packed and generally appeared within 3-6 weeks after initiation of the culture (~four to seven passages).

These ES cell-like colonies grew selectively with an increase in the number thereof when cultured on a mouse fetal fibroblast feeder using Dulbecco's modified Eagles medium supplemented with FCS, 2-mercaptoethanol, mouse leukemia inhibitory factor (LIF), and glial cell derived neurotrophic factor (GDNF). After two to three passages, most colonies in the culture consisted of these ES-like colonies (Figure 1c). These ES-like colonies could be maintained with standard ES cell culture conditions (culture on mouse embryonic fibroblast feeder cells using Dulbecco's modified Eagle's medium supplemented with FCS, 2-mercaptoethanol and mouse leukemia inhibitory factor (LIF)). The morphology of the cells did not change as long as the cells were maintained in ES cell culture conditions. In contrast, GS cells could not be propagated under these conditions due to the absence of GDNF, an essential growth factor for the self-renewing division of spermatogonial stem cells (Science, vol.287, p1489-1493, 2000).

Cytogenetic analysis by quinacrine plus Hoechst 33258 staining showed that the ES-like cells had a normal karyotype (40, XY) in 70%-85% of metaphase spreads (Figure 2).

ES-like cells could be propagated in vitro for more than 5 months with 30-48 passages while maintaining an undifferentiated state. These results were reproducible; because similar cells were obtained in four of 21 experiments from mice with a different genotype (ddY, DBA/2, ICR and the like) and ages (0 to 8 days).

The overall frequency of forming ES-like cells was 1 in 1.5 x 10⁷ cells (equivalent to 35 newborn testes). Significantly, neither GS nor ES-like cells appeared when newborn testis cells were cultured directly in ES culture conditions in at least 20 experiments. Likewise, neither GS nor ES-like cells appeared when neonatal testis cells were cultured in the presence of membrane bound Steel factor (mSCF), LIF, and bFGF (EG cell culture condition) in at least 15 experiments; the addition of GDNF was a prerequisite for the development of both GS and ES-like colonies.

To determine whether GS cells can convert to ES-like cells, the present inventors picked a total of 148 GS cell colonies by micromanipulation at 2 months after culture initiation. These GS cells were transferred to a 96-well plate and expanded for an additional 3 months. As a result, one GS cell colony produced ES-like cells. The pluripotency of the ES-like cells was confirmed by teratoma-forming capability in vivo with subcutaneous injection into nude mice.

In addition, the present inventors used p53 knockout mice (Oncogene, vol.8, p3313-3322, 1993). The p53 knockout mice have a high frequency of testicular teratoma (APMIS, vol.111, p184-191, 2003). The present inventors hypothesized that ES-like cells have a close relationship with teratoma-forming cells and asked whether established GS cells from this strain convert more easily to ES-like cells. GS cells were established from a newborn p53 knockout mouse in an ICR background. The growth speed and morphology of GS cells were indistinguishable from those of wild-type cells, and GDNF was similarly required to obtain GS cells.

Two months after culture, 30-40 GS cell colonies of undifferentiated morphology were picked by micromanipulation, transferred to a 96-well plate, and cultured in GS cell culture medium (containing GDNF, LIF, bFGF and EGF). Significantly, ES-like cells appeared in these GS cell-derived cultures in two separate experiments within 2 months, and the colonies were morphologically indistinguishable from ES-like colonies from wild-type cells.

The frequency of appearance of ES-like cells from P53 knockout GS cells (twice in the two experiments) was extremely higher than that with the use of wild type GS cells.

Using p53 knockout mice, the present inventors also examined whether GS cells from mature testis can produce ES-, like cells. Spermatogonial stem cells were collected from 3-to 8-week-old mice using anti-CD9 antibody and cultured in GS cell medium. GS cells developed in two to three experiments. GS cells of undifferentiated morphology were picked 4-7 days after culture initiation, and the colonies were expanded in vitro on mitomycin C-inactivated mouse embryonic fibroblast (MEF). In total, ES-like cells appeared in two of eight experiments within 4 weeks of culture.

To examine the phenotype of the ES-like cells, the present inventors established a culture from Green mice. Since these Green mice express the enhanced green fluorescence protein (EGFP) gene ubiquitously, including in spermatogenic cells (enhanced green fluorescent protein), cultured cells can be distinguished from feeder cells under excitation with UV light.

EGFP-positive cells (ES-like cells) out of the above-described cultured cells were analyzed for the expression of surface antigens by flowcytometer, and were shown to constitute nearly one phenotypical population (Figure 3).

As shown in Figure 3 (a) to (h), these cells were positive for SSEA-1 (ES cell marker) (Proc. Natl. Acad. Sci. USA, vol.75, p5565-5569, 1978), β1- and a6-integrin (ES and GS cell marker) (Biol. Reprod., vol.69, p612-616, 2003), EpCAM (ES and spermatogonia cell marker) (J. Reprod. Fertil., vol.116, p379-384, 1999), and CD9 (ES and spermatogonial stem (GS) cell marker) (Biol. Reprod., vol.70, p70-75, 2004), positive or weakly positive for EE2 (spermatogonia marker) (Mol. Reprod. Dev., vol.40, p221-227, 1995), and weakly positive for Forssman antigen (ES cell marker) (Nature, vol.292, p154-156, 1981) and c-kit (differentiated spermatogonia marker) (Endocrinology, vol.140, p5894-5900, 1999).

In contrast, GS cells were completely negative for SSEA-1 and Forssman antigen (Figure 4 (a) and (f)), suggesting that ES-like cells are phenotypically distinct from GS cells. In addition, GS cells were positive for β1- and a6-integrin, EpCAM, CD9, EE2 and c-kit (Figure 4 (b)-(e), (g) and (h)). GS cells from p53 knockout mice showed similar expression profile (data not shown).

In addition, ES cells were positive for SSEA-1, β1- and a6-integrin, EpCAM, CD9, Forssman antigen and c-kit, and weakly positive for EE2 (Figure 5 (a)-(h)).

Testis cells before the start of culture were negative for SSEA-1; about 40% of the population were positive for Forsman antigen (FIG. 6 (a) (b), FIG. 7). Although the present inventors found some expression of Forssman antigen in the neonatal testis cell population before culture, it was expressed by a non-germ cell population, and no EE2-positive cells were found (Figure 8).

The ES-like cells were also strongly positive for alkaline phosphatase, which is characteristic of ES cells (Figure 9 (a)). On the other hands, GS cells were weakly positive or negative for alkaline phosphatase (Figure 9 (b), suggesting that the ES-like cells have a distinct phenotype from GS cells. ES cells are positive for alkaline phosphatase (Figure 9 (c)).

Next, the present inventors used the reverse transcriptase-polymerase chain reaction (RT-PCR) to examine several molecules that are specifically expressed in embryonal carcinoma (EC) or ES cells. In addition to Oct-4, Rex-1, and Nanog, which are essential for maintaining undifferentiated ES cells (Stem Cells, vol.19, p271-278, 2001, Proc. Natl. Acad. Sci. USA, vol.100, p14926-14931, 2003, Cell, vol.113, p631-642, 2003, Cell, vol.113, p643-655, 2003), the ES-like cells expressed Cripto, ERas, UTF1, and ZFP57 at similar levels to ES cells (Dev. Biol., vol.235, 12-32, 2001; Nature, vol.423, 541-545, 2003; EMBO J., vol.17, p2019-2032, 1998; Genome Res., vol.12, 1921-1928, 2002; Dev. Biol., vol.265, 491-501, 2004). These results suggest that the ES-like cells are similar to ES cells in phenotype. GS cells also expressed some of these molecules, but the expression was generally weaker. , Significantly, present inventors could not detect expression of Nanog in GS cells, suggesting that GS cells have a different mechanism for self-renewal from that of ES cells ,and that the ES-like cells have different phenotypes from GS cells (Figure 10 and 11).

To analyze the imprinting pattern of ES-like cells, differentially methylated regions (DMRs) of three paternally imprinted regions (*H19, Meg3 IG*, and *Rasgrf1* regions) and two maternally imprinted regions (*Igf2r* and *Peg10* regions) were examined by bisulfite sequencing with two independent cells (Figure 12). While the paternally imprinted regions were methylated to different degrees, the maternally imprinted regions were rarely methylated in ES-like cells. DMRs in ES cells were generally more methylated than those in ES-like cells, including maternally imprinted regions, and the DMRs of the *H19* region were methylated more extensively than the DMRs of other regions. In contrast, GS cells showed a complete androgenetic imprinting pattern: the complete methylation of both the *H19* and *Meg3 IG* DMRs and demethylation of the *Igf2r* DMR.

Next, the present inventors examined the imprint status of GS or ES-like cells from p53 knockout mice. Genomic DNA was isolated from the same cell population at four different time points during the conversion of GS cells into ES-like cells. In these experiments, the imprint status in the DMRs was determined by combined bisulfite restriction analysis (COBRA) (Nucl. Acid. Res., vol.25, p2532-2534, 1997)(Figure 13A). As expected from the analysis of wild-type GS cells, GS cells from p53 KO mice had an androgenetic imprint pattern. However, a loss of methylation in the DMRs of H19, Meg 3IG, and Rasgrf1 regions and methylation of the DMRs in the Igf2r region were observed immediately after the appearance of ES-like cells. The perturbation of imprint patterns continued even when GS cells disappeared, and only the DMR of the Peg10 region was intact, 18 days after the appearance of ES-like cells. DMR of Oct-4 region in ES and ES-like cells were all hypomethylated, which confirms their undifferentiated state (J. Biol. Chem., vol.279, p17063-17069, 2004)(Figure 13B).

To determine whether ES-like cells can differentiate into somatic cell lineages, the present inventors used methods designed to induce differentiation of ES cells in vitro. ES-like cells derived from Green mice were first transferred to an OP9 stromal feeder layer. The OP9 stromal feeder cells can support differentiation of mesodermal cells such as hematopoietic, blood or muscle cells (Science, vol.265, p1098-1101, 1994; Proc. Natl. Acad. Sci. USA, vol.100, p4018-4023, 2003). Within 10 days, a variety of cell types were identified including hematopoietic cells, blood cells, vascular cells (endothelial cells and the like) (CD31 positive cells), and spontaneously beating myocytes (MF20 positive cells). The blood cell system cells comprised erythroblasts (Ter119-positive cells), blood cells (CD45-positive cells), myeloid system cells [myeloid precursor cells, monocytic cells (Mac1-positive cells), and neutrophilic cells (Gr1-positive cells)] (FIG. 14A-H, FIG. 15).

Hematopoiesis could also be induced when ES-like cells were cultured in methylcellulose to form embryoid bodies (Figure 14I). When ES-like cells were transferred onto gelatin-coated dishes for the differentiation of neural-lineage cells (Nat. Biotech., vol.21, p183-186, 2003), they formed neurons (MAP2 positive cells) or glial cells (MBP positive cells) (Figure 14 J-L). Dopaminergic neurons were also found, albeit at low frequency (Figure 14M). When the present inventors compared the differentiation efficiency using ES cells, ES-like cells produced more glial cells than did ES cells, and there were significantly more vessel cell (endothelial cell and the like) or heart muscle cell colonies from ES-like cells. However, ES-like cells could produce all of the expected lineages using protocols for ES cell differentiation (Table 1). ,

**Table 1**

| | Hematopoiesis*† | | | Vasculogenesis*‡ | | Neurogenesis§ | | |
|---|---|---|---|---|---|---|---|---|
| Cell type | Increase in cell number (fold) | Granulocyte /Macrophage (%) | Erythrocyte¶ (%) | Vesse¶ | Heart¶ | Neuron¶ | Astrocyte¶ | Oligodendrocyte¶ |
| ES-like | 116.7 15.4 | 7.6 0.2 | 19.9 0.7 | 111.5 12.0 | 8.0 4.5 | 126.7 14.4 | 34.6 4.4 | 4.6 2.5 |
| ES cell | 102.3 11.6 | 7.6 0.4 | 24.7 0.9 | 49.0 9.2 | 3.8 2.0 | 162.2 14.5 | 10.5 3.3 | 0.2 0.1 |

Table 1 shows in Vitro Differentiation of ES-Like Cells from Testis. Values in the table are mean ± SEM. Results from at least three experiments. ES cells were derived from 129 mice, whereas ES-like cells were derived from DBA/2 mice. *: Flk-1-positive cells (5 10³) were sorted, 4 days after co-culture and replated on OP9 feeder in 24-well plate. ^{†}: Cells were recovered 7 days after sorting and analyzed by flow cytometry. Erythrocytes, macrophages, and granulocytes were identified by anti-Ter119, anti-Mac1, and anti-Gr1 antibodies, respectively. ^{‡}: Numbers of positive cells in each well, 8 days after sorting. Vascular cells were determined by the uptake of DiI-acetylated low-density lipoprotein. Heart muscle colonies were identified by counting beating colonies. ^{§}: Cells (2.5 10⁴) were plated on gelatin in 48-well plate, and numbers of positive cells per one cm² were determined, 5 (neuron) or 7 (astrocytes or oligodendrocytes) days after plating. Neurons were identified by anti-Tuj antibody, whereas astrocytes and oligodendrocytes were identified by anti-GFAP or anti-MBP antibodies, respectively. Dopaminergic neurons were produced - 10 cells/well. ^{¶}: Statistically significant by t-test (P < 0.05).

ES-like cells were further examined for their ability to form teratomas in vivo by subcutaneous injection into nude mice. Transplanted cells gave rise to typical teratomas in all recipients (8/8) by 3 or 4 weeks after transplantation (Figure 14N). The tumors (teratomas) contained derivatives of the three embryonic germ layers: neuron, epidermis, muscle, bronchial epithelium, cartilage, bone, squamous cell epithelium, neuroepithelium, and the like. Similar results were obtained with three different clones or with ES-like cells from p53 knockout mice (8/8), and the present inventors did not observe a significant histological difference from teratomas derived from ES cells. In contrast, no tumors developed after subcutaneous transplantation of GS cells or fresh testis cells into nude mice (data not shown). Therefore, it was shown that the ES-like cells have the characteristic of differentiating into diverse somatic cell lineage in a manner similar to that for ES cells.

Since the ES-like cells originated from testis, their ability to differentiate into germline cells was examined using the spermatogonial transplantation technique (Proc. Natl. Acad. Sci. USA, vol.91, p11298-11302, 1994; JP-A-7-501705). This method allows spermatogonial stem cells to recolonize the empty seminiferous tubules of infertile animals and differentiate into mature sperm. We transplanted the cultured cells into immune-suppressed immature W mice (Biol. Reprod., vol.68, p167-173, 2003). These mice are congenitally infertile and have no differentiating germ cells (Proc. Natl. Acad. Sci. USA, vol.91, p11298-11302, 1994). One month after transplantation, all recipient animals (10/10) developed teratomas in the testis. The seminiferous tubules were disorganized, and no sign of spermatogenesis was found in histological sections. The cell composition found in the teratomas was similar to that of tumors that developed after subcutaneous injection (data not shown); this shows that the microenvironment of the seminiferous tubule does not influence the differentiation pattern of the cultured cells. In contrast, both wild-type and p53 KO GS cells produced normal spermatogenesis within 2 months after transplantation when transplanted into the seminiferous tubules (Figure 14 O-Q).

Additionally, the present inventors microinjected ES-like cells into blastocysts to examine differentiation properties of the ES-like cells in vivo. This is because ES cells colonize in blastocysts and contribute to all cell types in the body, including germline. Five to fifteen ES-like cells derived from Green mice were injected into C57BL/6 blastocysts. The ratio of euploid cells, which significantly influences the rate of chimerism or germline transmission (Transgenic Res., vol.6, p321-328, 1997), was 70% at the time of injection.

After being cultured in vitro for 24 hours, the chimeric embryos were returned to the uterus of pseudo-pregnant recipient mice. Some of the recipient animals were analyzed for chimerism at 12.5 dpc, and the others were allowed to develop to term. At 12.5 days of viviparity, fetuses had developed normally, with chimerism observed in 25% (3/12) thereof, and the expression of EGFP was observed in the whole body of each fetus under UV light (FIG. 16A). The chimeric mouse fetuses were born by spontaneous delivery; chimerism was observed in 36% (13/36) of the newborn animals as in the fetuses (FIG. 16B) (FIG. 17). Chimerism was also confirmed by the coat color at mature stage (Figure 16C). The present inventors found six dead fetuses that showed EGFP expression, and some embryos were partially or completely absorbed. The pattern of contribution of donor cells was similar at both stages analyzed (embryos and newborn animals); EGFP-positive donor cells were found in the central nervous system (brain, bone marrow, neural tube and the like), liver, heart, lung,, testis, somites, intestine, and other tissues, including the yolk sac and chorionic membrane of the placenta (Figure 16 D-J, Figure 18).

Since donor cells were also found in the testis of a chimera mouse at 6 weeks of age, microinsemination was performed to obtain offspring. Round spermatids were collected and microinjected into C57BL/6 × DBA/2 (BDF1) oocytes. Of 81 cultured embryos, 64 (79%) developed into 2-cells and were transferred into five pseudopregnant females. Eighteen (22%) embryos were implanted, and one of the two offspring from a recipient mouse showed EGFP fluorescence, indicating the donor origin (Figure 16 K). Interestingly, while control ES cells showed wide contribution to embryos, no donor cell contribution was observed in experiments using GS cells (Table 2).

**Table 2**

| Type of cells | Number of embryos transferred | Number of recipients | Number of pups born * | Number of live pups† | Chimera (%) | |
|---|---|---|---|---|---|---|
| | | | | | Male | Female |
| ES-like | 193 | 11 | 54 | 36 | 9/22 (41) | 4/14 (29) |
| ES | 91 | 14 | 14 | 4 | 2/2 (100) | 2/2 (100) |
| GS | 124 | 7 | 28 | 16 | 0/8 (0) | 0/8 (0) |
| 4n rescue ES-like | 92 | 4 | 0 | NA | NA | NA |
| 4n rescue ES | 30 | 2 | 0 | NA | NA | NA ' |

Table 2 represents contribution of ES-like cells to embryonic development. NA: not applicable. *: In some experiments, fetuses were delivered by cesarean section at 19.5 dpc. ^{†}: Number of live pups on the next day after birth. To determine the full developmental potential of ES-like cells, the present inventors used tetraploid complementation technique (Proc. Natl. Acad. Sci. USA, vol.90, p8424-8428, 1993). This technique allows the production of live animals that consist entirely of donor ES cells. A total of 92 tetraploid embryos were created by electrofusion, aggregated with ES-like cells, and transferred to pseudopregnant ICR females. When some of the recipient animals were sacrificed at 10.5 dpc, one normal-looking fetus and several resorptions with normal placentas were found. The fetus showed some growth retardation but clearly expressed the EGFP gene throughout its body, including the yolk sac (Figure 16 L), indicating that the fetus was derived from donor ES-like cells.

Hence, it was shown that the ES-like cells of the present invention have the capability of differentiating into all somatic cells, including germline (totipotency) in vivo as well.

### (Discussions)

The results of the above mentioned experiments revealed the presence of multipotential stem cells in the postnatal testis. Although some cases of the "stem cell plasticity" phenomenon have been attributed to cell fusion (Cell, vol.116, p639-648, 2004), the ES-like cells of the present invention cannot be explained by the same mechanism because the ES-like cells of the present invention formed teratomas after subcutaneous transplantation. These ES-like cells from the testis can be considered the postnatal counterparts of ES/EG cells. The results of the Example were unexpected, since PGCs become resistant to experimental teratocarcinogenesis or EG cell formation after 13.5 dpc (Cell Differ., vol.15, p69-74, 1984; Development, vol.120, p3197-3204, 1994). EG cells are considered to be the only example of the isolation of multipotent stem cells from primary germ cells (Nature, vol.359, p550-551, 1992; Cell, vol.70, p841-847, 1992). EG cells were derived from primary germ cells harvested from 8.5 to 12.5 dpc fetuses and cultured in vitro with a mixture of mSCF, LIF, and bFGF. However, pluripotent cells could not be isolated from neonatal germ cells using the same culture conditions (Development, vol.120, p3197-3204, 1994), except when cells after in vivo teratoma formation were cultured. ES-like cells of the present invention are unlikely to be derived from teratoma cells for two reasons. First, the frequency of derivation of ES-like cells in the present invention was significantly higher than the negligible rate of spontaneous teratoma formation (one teratoma out of 11,292 males in 129 hybrid backgrounds) (J. Natl. Cancer. Inst., vol.27, p443-453, 1961). Second, growth factor supplementation was essential for the establishment of ES-like cells. In fact, few EC cell lines have been obtained from spontaneously occurring teratocarcinomas (Experimental approaches to mammalian embryonic development, Cambridge University Press, p475-508, 1986). Therefore, the ability to become multipotent stem cells may persist in neonatal testis. Based on the Example, the present inventors propose to name the ES-like cells of the present invention multipotent germline stem cells, or mGS cells, to distinguish them from GS cells, which can differentiate only into germline cells).

An important question that arises from this invention is the origin of mGS cells. One possibility is that mGS cells appear independently from GS cells and originate from a population of undifferentiated pluripotent cells that persist in the testis from the fetal stage. Although EG cells have been established from -12.5 dpc PGCs (Cell, vol.70, p841-847, 1992; Development, vol.120, p3197-3204, 1994), cells with similar characteristics might remain in neonatal testis and produce ES-like cells. Indeed, the results of the imprinting analysis of wild-type mGS cells suggest a distinct origin for mGS cells. In male germ cells, genomic imprinting is erased during the fetal stage, and male-specific imprinting begins to be acquired around birth in prospermatogonia and is completed after birth (Genomics, vol.58, p18-28, 1999; Hum. Mol. Genet., vol.9, p2885-2894, 2000; Genes Dev., vol.6, p705-714, 1992). While GS cells had a typical androgenetic imprinting pattern, the imprinting pattern of mGS cells clearly differed from those of androgenetic germ cells or somatic cells, suggesting that mGS cells can originate from partially androgenetic germ cells that have undergone imprint erasure.

Gonocytes in the testes of newborns have been reported to be heterogenous; pseudopod gonocytes have the capability of colonizing spermatogenesis after spermatogonial transplantation, whereas round gonocytes do not colonize spermatogenesis but undergoes apoptosis in vitro. Because mGS cells differ from GS cells in terms of spermatogonial stem cell activity, mGS and GS cells may have originated from different types of gonocytes.

Another possibility is that mGS cells are derived from spermatogonial stem cells and that the ability to become multipotential cells may be one of the general characteristics of germline cells (spermatogonial stem cells and the like). Possibly, the interaction with Sertoli cells normally directs germ cells to spermatogenesis and inhibits multilineage differentiation in the testis. However, when germline cells are continuously stimulated to expand in the absence of Sertoli cells, in the culture conditions of the present invention, germ cells may be released from this inhibition and some of the cells converted to pluripotent cells. Teratogenesis is susceptible to environmental influences; teratoma formation can be significantly enhanced (-10-fold) in vivo by ectopic transplantation of the fetal genital ridge (Cell Dev., vol.15, p69-74, 1984). In the method of the present invention of , producing pluripotent stem cells, the environment in the testis seems to be suppressive on multilineage differentiation because dilution of somatic cells by passage at an early stage after the start of culture is effective in establishing mGS cells. As PGCs can become pluripotential only after in vitro culture and cytokine supplementation was also necessary for EG cell conversion (Cell, vol.70, p841-847, 1992; Nature, vol.359, p550-551, 1992), growth stimulation and release from somatic cells may modify the differentiation program of germline cells.

Several lines of evidence in the Example provide support for the multipotential nature of spermatogonial stem cells. First, PGC-like germ cells were not found in the neonatal testis, and mGS cells could not be induced from neonatal testis in EG cell culture conditions (mSCF + LIF + bFGF). Therefore, the mGS cells arose through a different mechanism from that of EG cells, and the results suggest that PGC-like cells in neonatal testis, if any, are not responsible for the generation of mGS cells.

Second, the result that mGS cells emerged from cells from among picked up GS cell colonies derived from wild type and P53 knockout mice means that mGS cells develop from GS cells. Loss of the p53 gene results in a 100-fold increase in the susceptibility to testicular teratoma formation (APMIS, vol.111, p184-191, 2003). Nevertheless, GS cells from this strain were phenotypically similar to wild-type spermatogonia and could produce normal-appearing spermatogenesis when transferred into seminiferous tubules. In this sense, GS cells from P53 knockout mice are indistinguishable from wild-type GS cells and fulfill the criteria for spermatogonial stem cells. Using this model, the present inventors found that the partial androgenetic imprint in mGS cells occurred with loss of the androgenetic imprint in GS cells. Perhaps the same is true of wild-type mGS cells; the partial androgenetic imprint patterns may not indicate the origin of mGS cells directly but rather reflect epigenetic instability in vitro, as reported for ES/EG cells (Development, vol.120, p3197-3204, 1994; Development, vol.125, p2273-2282, 1998; Science, vol.293, p95-97, 2001).

These results strongly suggest that GS cells are multipotential or can easily acquire multipotentiality by loss of a single gene (P53). Teratoma formation in mice occurs almost exclusively in the 129/Sv background and is considered to develop from PGCs (Cell Differ., vol.15, p69-74, 1984). However, the above-mentioned Example strongly suggests that spermatogonial stem cells are multipotential.

Interestingly, the acquisition of multipotentiality in mGS cells was concurrent with the loss of spermatogonial stem cell potential. Despite their testicular origin, mGS cells formed teratomas when transferred in the seminiferous tubules, indicating that this environment was no longer able to support germ cell development (spermatogenesis) after the cells became pluripotent. This contrasts with GS cells, which produce spermatogenesis after long term cultivation (Biol. Reprod., vol.69, p612-616, 2003). Therefore, mGS cells are more closely related to ES/EG cells in terms of cell function. The reason for the loss of spermatogonial stem cell potential is unknown; however, the present inventors speculate that it may be related to the loss of responsiveness to GDNF during the course of the establishment of mGS cells, as GDNF is an essential factor for promoting the self-renewing division of spermatogonial stem cells in vivo (Science, vol.278, p1489-1493, 2000).

One of the most important results from the above-mentioned Experiment is the contribution of mGS cells to normal embryo development. Donor cell makers were present in various parts of the body, including the germline cells. These results demonstrate that mGS cells not only produce tumors but also can contribute to normal embryonic development. The imprint status of mGS cells did not influence the germline competence, and normal offspring were obtained from the chimeric animal. This agrees with the previous reports that both ES and EG cells can produce germline chimera, even with androgenetic imprint , patterns (Experimental approaches to mammalian embryonic development, Cambridge University Press, p475-508, 1986; Development, vol.120, p3197-3204, 1994; Dev. Biol., vol.161, p626-628, 1994; Curr. Biol., vol.7, p881-884, 1997).

The derivation of multipotent stem cells from the postnatal testis has important practical value for medicine and biotechnology. The mGS cells produced by the method of the present invention are different from other reported multipotent cells obtained from postnatal animals in terms of morphology, marker expression, and capacity for differentiation (Trends Cell Biol., vol.12, p502-508, 2002; Cell, vol.116, p639-648, 2004). While it is important to study the biology of individual cell types and assess their potential for clinical application, a major advantage of mGS cells is that techniques used to derive specific lineages of cells from ES cells are applicable directly. The derivation of mGS cells has fewer ethical concerns than does the derivation of ES cells, because mGS cells can be obtained from postnatal animals without sacrificing the animals (including conceptus or embryos). Furthermore, the availability of histocompatible, multipotent tissue for autotransplantation would circumvent immunological problems associated with ES cell-based technology. The results of the p53 knockout mouse experiment and the like suggest that mGS cells can arise from mature testis. Development of more efficient systems to derive GS cells from mature testis is important at this stage of research, and suppression of p53 expression in GS cells, such as by RNA interference, may be useful for enhancing the frequency of derivation of mGS cells. Studies directed toward examining the effect of imprinting on the range and efficiency of differentiation may be important.

Although postnatal male germ cells have been considered to be fully committed to produce sperm, the present invention demonstrates their pluripotentiality and also indicates that testis can serve as a source to derive ES-like stem cells. Together with GS cells, a new stem cell line described here has important implications in understanding the biology of germline and provides a unique tool for biotechnology and medicine.

### Industrial Applicability

Using the production method of the present invention, it is possible to produce pluripotent stem cells, which have conventionally been only obtainable from fertilized eggs, embryos and the like, from a postnatal individual. Using the pluripotent stem cells, it is possible to construct diverse tissues having histocompatibility for autotransplantation, and the pluripotent stem cells are useful in medical fields such as regeneration medicine and gene therapy. Also, the pluripotent stem cells are useful in the field of biotechnology because they can be used to prepare transgenic animals, knockout animals and the like.

This application is based on a patent application No. 2004-101320 filed in Japan (filing date: March 30, 2004), the contents of which are incorporated in full herein by this reference.

Sequence Listing Free Text
SEQ ID NO: 1: specific primer for Oct-4
SEQ ID NO: 2: specific primer for Oct-4
SEQ ID NO: 3: specific primer for UTF1
SEQ ID NO: 4: specific primer for UTF1
SEQ ID NO: 5: specific primer for HPRT
SEQ ID NO: 6: specific primer for HPRT
SEQ ID NO: 7: specific primer for H19
SEQ ID NO: 8: specific primer for H19
SEQ ID NO: 9: specific primer for Meg3 IG
SEQ ID NO: 10: specific primer for Meg3 IG
SEQ ID NO: 11: specific primer for Rasgrf1
SEQ ID NO: 12: specific primer for Rasgrf1
SEQ ID NO: 13: specific primer for Igf2r
SEQ ID NO: 14: specific primer for Igf2r
SEQ ID NO: 15: specific primer for Peg10
SEQ ID NO: 16: specific primer for Peg10
SEQ ID NO: 17: specific primer for Oct-4
SEQ ID NO: 18: specific primer for Oct-4

## Claims

1. A method of producing pluripotent stem cells, which comprises culturing testis cells using a medium containing glial cell derived neurotrophic factor (GDNF) or an equivalent thereto to obtain pluripotent stem cells.

2. The production method of claim 1, wherein the medium further contains leukemia inhibitory factor (LIF).

3. The production method of claim 1 or 2, wherein the medium further contains at least one of epidermal growth factor (EGF) and basic fibroblast growth factor (bFGF). ,

4. The production method of any one of claims 1 to 3, which comprises culturing testis cells in the presence of feeder cells.

5. The production method of claim 1, wherein the testis cells are spermatogonial stem cells.

6. The production method of claim 5, wherein the spermatogonial stem cells are GS cells.

7. The production method of claim 1, wherein the testis cells are P53-deficient.

8. The production method of claim 1, which comprises the following steps:
(Step 1) culturing testis cells using a medium containing glial cell derived neurotrophic factor (GDNF) or an equivalent thereto to obtain cultured cells;
(Step 2) culturing the cultured cells obtained in Step 1, using a medium containing leukemia inhibitory factor (LIF) to obtain pluripotent stem cells.

9. The production method of claim 8, wherein the medium for Step 1 further contains leukemia inhibitory factor (LIF).

10. The production method of claim 8 or 9, wherein the medium for Step 1 further contains at least one of epidermal growth factor (EGF) and basic fibroblast growth factor (bFGF).

11. The production method of any one of claims 8 to 10, wherein Step 1 comprises culturing testis cells in the presence of feeder cells.

12. The production method of claim 1, which comprises the , following steps:
(Step 1) culturing testis cells using a medium containing glial cell derived neurotrophic factor (GDNF) or an equivalent thereto to obtain GS cells;
(Step 2) culturing the GS cells obtained in Step 1, using a medium containing glial cell derived neurotrophic factor (GDNF) or an equivalent thereto to obtain pluripotent stem cells.

13. The production method of any one of claims 1 to 12, wherein the testis cells are derived from a mammal.

14. The production method of claim 13, wherein the mammal is postnatal.

15. The production method of claim 1, wherein the pluripotent stem cells are positive for at least any one selected from the group consisting of SSEA-1, Forsman antigen, β1-integrin, α6-integrin, EpCAM, CD9, EE2 and c-kit.

16. The production method of claim 15, wherein the pluripotent stem cells are positive for SSEA-1, Forsman antigen, β1-integrin, α6-integrin, EpCAM, CD9, EE2 and c-kit.

17. A pluripotent stem cell produced by the production method of any one of claims 1 to 16.

18. A pluripotent stem cell derived from a testis cell, which is positive for at least any one selected from the group consisting of SSEA-1, Forsman antigen, β1-integrin, α6-integrin, EpCAM, CD9, EE2 and c-kit.

19. The pluripotent stem cell of claim 18, which is positive for SSEA-1, Forsman antigen, β1-integrin, α6-integrin, EpCAM, CD9, EE2 and c-kit.

20. A method of producing a chimeric embryo, which comprises the following steps:
(Step 1) culturing testis cells using a medium containing glial cell derived neurotrophic factor (GDNF) or an equivalent thereto to obtain pluripotent stem cells;
(Step 2) introducing the pluripotent stem cells into a host embryo to obtain a chimeric embryo.

21. A method of producing a chimeric animal (excluding humans), which comprises the following steps:
(Step 1) culturing testis cells using a medium containing glial cell derived neurotrophic factor (GDNF) or an equivalent thereto to obtain pluripotent stem cells;
(Step 2) introducing the pluripotent stem cells into a host embryo to obtain a chimeric embryo;
(Step 3) transferring the chimeric embryo to the uterus or oviduct of a host animal to obtain a chimeric animal (excluding humans).

22. A method of producing a non-human animal derived from pluripotent stem cells, which comprises the following steps:
(Step 1) culturing testis cells using a medium containing glial cell derived neurotrophic factor (GDNF) or an equivalent thereto to obtain pluripotent stem cells;
(Step 2) introducing the pluripotent stem cells into a host embryo to obtain a chimeric embryo;
(Step 3) transferring the chimeric embryo to the uterus of a host animal to obtain a chimeric animal (excluding humans); (Step 4) mating the chimeric animal to obtain a non-human animal derived from the pluripotent stem cells.

23. A method of producing a tetraploid chimeric embryo, which comprises the following steps:
(Step 1) culturing testis cells using a medium containing glial cell derived neurotrophic factor (GDNF) or an equivalent , thereto to obtain pluripotent stem cells;
(Step 2) introducing the pluripotent stem cells into a tetraploid embryo to obtain a tetraploid chimeric embryo.

24. A method of producing a non-human animal derived from pluripotent stem cells, which comprises the following steps:
(Step 1) culturing testis cells using a medium containing glial cell derived neurotrophic factor (GDNF) or an equivalent thereto to obtain pluripotent stem cells;
(Step 2) introducing the pluripotent stem cell into a tetraploid embryo to obtain a tetraploid chimeric embryo;
(Step 3) transferring the tetraploid chimeric embryo to the uterus or oviduct of a host animal to obtain a non-human animal derived from the pluripotent stem cells.

25. A method of producing functional cells, which comprises the following steps:
(Step 1) culturing testis cells using a medium containing glial cell derived neurotrophic factor (GDNF) or an equivalent thereto to obtain pluripotent stem cells;
(Step 2) culturing the pluripotent stem cells under functional cell differentiation conditions to obtain functional cells.

26. The production method of claim 25, wherein the functional cells are mesodermal cells.

27. The production method of claim 26, wherein the mesodermal cells are any one selected from the group consisting of blood cell lineage cells, vascular lineage cells and myocardial cells.

28. The production method of claim 25, wherein the functional cells are ectodermal cells.

29. The production method of claim 28, wherein the ectodermal cells are neuronal lineage cells.

30. The method of claim 29, wherein the neuronal lineage cells are any one selected from the group consisting of neurons, glial cells, oligodendrocytes and astrocytes.

31. The production method of claim 25, wherein the functional cells are endodermal cells.

32. A composition for producing pluripotent stem cells derived from a testis cell, which contains glial cell derived neurotrophic factor (GDNF) or an equivalent thereto.

33. The composition of claim 32, which further contains leukemia inhibitory factor (LIF).

34. The composition of claim 32 or 33, which further contains at least one of epidermal growth factor (EGF) and basic fibroblast growth factor (bFGF).
